# EUROPEAN PATENT APPLICATION

(11) **EP 4 492 061 A1**
(43) Date of publication of application: **15.01.2025**
(21) Application number: 24160720.9
(22) Date of filing: 29.02.2024
(51) Int. Cl.: G01N 33/68, C07K 14/705, C07K 16/28, C12N 5/071

(54) **SEX-SPECIFIC SPERM MEMBRANE PROTEINS FOR SPERM SEXING**

(30) Priority: 11.07.2023 PT 2023118813; 05.01.2024 PT 2024119200
(71) Applicant: Universidade de Trás-os-Montes e Alto Douro, 5001-801 Vila Real (PT)
(72) Inventor: ANTUNES COLAÇO, BRUNO JORGE, 5000-801 VILA REAL (PT); PINTO DE PINHO, PATRÍCIA, 4890-283 CELORICO DE BASTO (PT); LEAL FREIRE QUELHAS REIS, JOANA, 4250-050 PORTO (PT); SÂNCIO DA CRUZ FARDILHA, MARGARIDA, 3810-123 AVEIRO (PT)
(74) Representative: Pereira da Cruz, Joao

(57) **Abstract**

The present disclosure relates to sperm sexing techniques, particularly to methods for the identification of potential membrane proteins that could serve as markers to distinguish X-sperm from Y-sperm. This enables their selective separation and further utilization in immunologically based sperm sexing techniques. Additionally, methods for protein analysis are provided.

## Description

### Background of the Disclosure

The present disclosure relates to the field of reproductive technologies, particularly to the field of sperm sexing techniques.

The present disclosure describes methods for the identification of membrane protein targets for sperm sexing technologies.

It is noteworthy that the global demand for food has been increasing along with the world population, which recently reached 8 billion people and is expected to continue to increase [1, 2]. In particular, there is a growing need for animal-sourced foods, such as meat, eggs, and milk, which are rich in animal protein and other indispensable nutrients and minerals [2-4]. Thereby, novel solutions are urgently required to achieve a more sustainable animal production that respects both animal rights and the environment, while also being economically viable [3, 5]. Some industries are mainly interested in one of the sexes; for example, females are more relevant than males for milk-producing companies. The same is true for other types of production, such as cuniculture aimed at females' selection for breeding purposes or multiplier farms that produce replacement gilts. In these cases, the undesired animals are frequently euthanized or sold at a low cost. This not only raises questions about animal welfare but is also undesirable from an economic perspective [2, 5].

To address this issue, several animal reproductive management techniques emerged throughout the years. Sperm sexing arose a few decades ago as a promising tool to easily and inexpensively pre-select the sex of animals, preventing many animals of the undesired sex to be unnecessarily slaughtered [6-8]. Overall, sperm sexing consists of the separation of a semen sample into two fractions, one carrying a large majority of X chromosome-bearing spermatozoa (X-sperm) and the other holding a large majority of Y chromosome-bearing spermatozoa (Y-sperm) [7, 9]. Currently, the most common method of sperm sorting is through flow cytometry, a disclosure patented in 1991 (Patent No. US5135759) by the United States Department of Agriculture [10]. This methodology is based on the differences in DNA content between the X and Y chromosomes, with X-sperm containing more DNA. By exposing spermatozoa to a fluorescent dye that binds to intact DNA, X-sperm will emit a brighter fluorescence than Y-sperm, allowing their differentiation and subsequent sorting [11-13]. Semen straws with 2 million sexed spermatozoa are widely commercialized with over 85-95% accuracy, and straws with 4 million are also available under the trade name SexedULTRA 4M, offering an accuracy of 90% or higher [14]. Nevertheless, despite years of efforts to improve semen sexing through flow cytometry, this technique negatively affects sperm quality and is still time-consuming and expensive [8, 13, 15].

Besides flow cytometry, other attempts at sperm sexing were performed, namely based on X- and Y- differential density [16, 17], electrophoresis separation [18], albumin density gradient [19], swim-up and Percoll density gradient (Patent No. WO2004072220A2) [20], and Raman spectroscopy [21]. Until now, neither of these techniques was successful, achieving a 75% accuracy at most and/or still requiring optimization and cost reduction to be widely implemented [22]. Sexcel Sexed Genetics^{™} commercialized by ABS Global company distinguishes itself from the aforementioned techniques, since it offers X-sorted semen for cattle with an accuracy of around 85%, based on the laser ablation of Y-sperm. Theoretically, compared to flow cytometry, it affects sperm quality less because it does not divide cells into droplets and does not require cell steering or an electric field [23-25].

More recently, immunological methods have emerged, based on the differential genomic content of X and Y chromosomes, which translates into different protein expression in X- and Y-sperm [22, 26, 27]. Umehara and colleagues developed a sexing method based on the activation of Toll-like receptor 7/8 (TLR 7/8). Both TLR7 and TLR8 were detected in the tail and midpiece, respectively, only in X-sperm. They were able to reduce X-bearing sperm motility, allowing the separation of X-sperm (lower layer) and Y-sperm (upper layer) [28].

Furthermore, there is increasing evidence that the surface of X- and Y- sperm is different regarding their protein content. Resorting largely to proteomic analysis, several authors have attempted to uncover these differences, hoping to find specific proteins for either type of spermatozoa [26, 27]. These membrane targets can be employed with immunological techniques to avoid some of the limitations of earlier approaches in sperm sexing. Once a specific protein unique to X- or Y-sperm has been identified, the X and Y fractions can be separated by using, for example, magnetic beads coated with an antibody specific to that protein. This involves incubating the semen sample with the coated beads, allowing the beads to recognize and bind to the target protein, and then using a magnet to isolate the X and Y fractions [26, 27]. Patent No. KR20180054197A discloses a sperm sexing apparatus based on magnetic beads.

Membrane targets, as described for some species can be used for sperm sexing (Patent No. WO2009014456 and Patent No. US20090208977A1), if a specific antibody is coupled, for example, to magnetic beads. This enables the recognition of the defined target, thus allowing the separation of X- and Y- sperm using a magnet. The binding agent can also be attached to a solid surface, allowing the target sperm to bind and remain attached to it.

The sperm sexing kit WholeMom (Nuri Science Inc.), recently available in the market, is an immunological method based on the addition of an antibody to the semen sample that promotes the agglutination of the Y-sperm to originate female gestations (Patent No. US10550177B2). It is commercially available but only for bovine, swine, goat, sheep, equine, and canine semen. A sperm sexing kit marketized by Nuri Science Inc under the name WholeMom is available for bovine, swine, goat, sheep, equine, and canine semen). By adding an antibody to the semen sample, Y-sperm agglutinates with a precision ranging from 70-85% depending on the species. This allows the gestation of only female animals. They also have a WholeMan kit for bulls that improves the motility of the Y-sperm compared to the X-sperm, allowing mainly male gestations [29, 30]. EMLAB Genetics also offers kits for sexing sperm of the same six species; it innovates by having kits for both X- and Y-sperm fertility and motility enhancement [31]. This allows the sperm to be sorted in the reproductive tract and more eggs to be fertilized with the sperm of interest, with an accuracy ranging from 65% to 90%, depending on the species and type of semen used (fresh, cooled, or post-thaw semen) [31].

Despite extensive research and experimentation, which resulted in the sperm sexing methodologies presented herein, there are few profitable sperm sexing options available for most livestock industries, including rabbits. As a result, there is a continued need for new, fast, inexpensive, and easy methods that do not negatively impact sperm quality, conception rates, or offspring health. Immunological-based methods are currently one of the most promising options and further research into the spermatozoa surface proteome holds great value in efficiently separating X- and Y-sperm. Such methodology would greatly benefit companies in the field, contributing to a more sustainable exploration of animal-derived products while also preventing financial loss.

Among some of the main species for meat consumption, such as cattle, goat, pig, sheep, chicken, and turkey, chickens stand out massively, with about 70.8 billion slaughtered for meat in 2020, according to the Food and Agriculture Organization of the United Nations. Pigs come next (~1.5 billion), followed by rabbits (~639.8 million). When it comes to meat consumption in tonnes, between 2000 and 2020, pigs and chickens once again stood out, followed by cattle.

Despite those recent options, there is no profitable sperm sexing technology applicable to most livestock industries. The rabbit production industry would benefit from sex pre-selection technologies, enabling X-sperm selection for breeding purposes and hybrid producing systems.

Therefore, based on the existing limitations in sexing technology, particularly for rabbits despite its commercial value, the present disclosure aims to identify proteins specific of X- and Y-sperm membranes, particularly in rabbits and cattle due to their commercial value.

A bioinformatic analysis was carried out to predict the utility of the protein targets identified in the present disclosure for other commercially relevant species, namely for swine, ovine, and equine. It is also a goal of the present disclosure to analyze if there are common targets between all species, which could corroborate the application of the present technique in other species.

### General Description of the Disclosure

The present disclosure aims to identify selected biological targets for sexing rabbit, bovine, and other mammalian semen for which the proteins are similar enough to integrate an immunologically based sperm sexing methodology.

The innovative factor is the fact that some of the proteins identified in the present disclosure have never been described before as possible markers for X- or Y-sperm, which will bring new sexing options, plausible to be extended to other species.

In an embodiment, the present disclosure refers to a method for in vitro identification of sperm sexing protein targets, preferably membrane targets, comprising the steps of:
collecting a semen sample from a mammal, preferably a human, rabbit, cattle, pig, sheep or horse; extracting proteins from the semen sample;
carrying out liquid chromatography with tandem mass spectrometry combined with liquid chromatography in said proteins (having the technical effect of separating the proteins before they ate analysed by the spectrophotometer increasing the quality and reliability of the results), optionally carrying out a treatment with PGNase (imparts the technical advantage of analysizing the glycoprotein fraction of the proteins) and optionally purifying and cleaning said proteins (imparts the technical advantage of removing contaminants and excess of reagents which can interfere in the subsequent analysis of the proteins);
performing a bioinformatic analysis of said sample to identify potential protein candidates for an immunologically based sperm sexing technique.

In an embodiment, the present disclosure refers to a method, wherein the protein targets have at least 95% homology with one or a combination of the following SEQ ID No. 1 (Adhesion G protein-coupled receptor G2 (ADGRG2)), SEQ ID No. 2 (Renin receptor (ATP6AP2)), SEQ ID No. 3 (V-set and immunoglobulin domain-containing protein 1 (VSIG1)), SEQ ID No. 4 (B-cell receptor-associated protein (BCAP31)), SEQ ID No. 5 (membrane-associated progesterone receptor component 1 (PGRMC1)), SEQ ID No. 6 (Four and a half LIM domains 1 (FHL1)), SEQ ID No. 7 (Plastin 3 (PLS3)), SEQ ID No. 8 (RNA helicase (DDX3X)), SEQ ID No. 9 (Disintegrin and metalloproteinase domain-containing protein 2 (ADAM 2)), SEQ ID No. 10 (Phospholipid-transporting ATPase IG (ATP11C)), SEQ No. 11 (Desmoglein-1 (DG1)), SEQ ID No. 12 (Monocarboxylate transporter 1 (MCT 1)), SEQ ID No. 13 (Plasma membrane calcium-transporting ATPase 4 (PMCA4)), SEQ ID No. 14 (A-kinase anchoring protein 4 (AKAP4)), SEQ ID No. 15 (Cylicin-1 (CYLC1)), SEQ ID No. 16 (FUN14 domain-containing protein 2 (FUNDC2)), SEQ ID No. 17 (Serine/threonine-protein phosphatase with EF-hands (PPEF1)), SEQ ID No. 18 (Alpha-enolase (ENO1)), SEQ ID No. 19 (GLIPR1-like protein 1 (GLIPR1L1)), SEQ ID No. 20 (Heat shock 70 kDa protein 1-like (HSPA1L)), SEQ ID No. 21 (Heat shock cognate 71 kDa protein (HSPA8)), SEQ ID No. 22 (Malate dehydrogenase, mitochondrial (MDH2)), SEQ ID No. 23 (Phosphatidylethanolamine-binding protein 1 (PEBP1)), SEQ ID No. 24 (Sodium/potassium-transporting ATPase subunit beta-3 (ATPB-3)), SEQ ID No. 25 (ATP synthase subunit beta, mitochondrial (ATP5F1B)), SEQ ID No. 26 (Heat shock-related 70 kDa protein 2 (HSP70.3)), SEQ ID No. 27 (Endoplasmic reticulum chaperone BiP (BiP)), SEQ ID No. 28 (Chaperonin 60 (CPN60)), SEQ ID No. 29 (ADAM metallopeptidase domain 20), SEQ ID No. 30 (Disintegrin and metalloproteinase domain-containing protein 1a-like), SEQ ID No. 31 (Hyaluronidase (SPAM1)), SEQ ID No. 32 (Alkaline phosphatase, tissue-nonspecific isozyme (AP-TNAP)), SEQ ID No. 33 (Heat shock 70 kDa protein 1A (HSPA1A)), and SEQ ID No. 34 (Sperm acrosome membrane-associated protein 4 (SPACA4)), preferably ATP11C, MCT1, ADAM2 and PMCA4.

In an embodiment, the present disclosure refers to a method having at least 98% homology with SEQ ID No 1 - 34, even more preferably having at least 99% homology with SEQ ID No 1 - 34.

In an embodiment, the present disclosure refers to the use of the method herein disclosed for in vitro sperm sexing technologies.

In an embodiment, the present disclosure refers to the use of the method herein disclosed in medicine, veterinary medicine and agriculture.

In an additional embodiment it is also within the scope of the present disclosure, after protein targets are identified, a method for *in vitro* sperm sexing comprising the step of incubating semen samples with magnetic beads or glass microbubbles coated with antibodies coated in antibodies targeting proteins specific to X- or Y-sperm. Other variations of the technique using beads are available, including the use of glass microbubbles coated with antibodies capable of gently floating upwards after binding to the cells. This eliminates the need for a magnetic field and is described to be beneficial when working with fragile cells or sorting high-volume samples.

### Brief Description of Figures

The following figures are shown as a representation of particular embodiments and shall not be regarded as limiting the scope of the disclosure.
**Figure 1****.** Data extraction pipeline.
**Figure 2****.** Protein sequence similarity (%) between the 49 human targets that were identified in the list of rabbit targets and the respective rabbit protein with the same gene and protein name.
**Figure 3****.** Protein sequence similarity (%) between 66 human X-targets identified in the rabbit proteome (which were not a priori present or characterized in the list of rabbit X-targets) and the corresponding rabbit proteins with the same gene and protein names. Gene names encoding proteins with less than 70% similarity between humans and rabbits are highlighted in grey. The chromosome associated with each rabbit protein is specified in parentheses next to the gene name - (X) X chromosome and (U) Unplaced.
**Figure 4****.** Liquid chromatography chromatograms of the rabbit unsexed semen samples and the negative controls that were not incubated with biotin.
**Figure 5****.** Schematization of the functional annotation of the quantified proteins using UniProt information and eggNOG-mapper v2.1.9 annotation, concerning entry status, protein name, gene name, and gene ontology identifiers (GO ID).
**Figure 6****.** Liquid chromatography chromatograms of the bovine unsexed semen, X-semen, Y-semen, and negative controls that were not incubated with biotin.
**Figure 7****.** Volcano plot of the fold change (in log2, on the X-axis) of each protein and the statistical significance (-log10 adjusted p-value on the Y-axis), between (A) bovine unsexed and bovine X-semen proteins; (B) bovine unsexed and bovine Y-semen proteins; and (C) bovine X-semen and bovine Y-semen proteins.
**Figure 8****.** Schematization of the functional annotation of the quantified proteins using UniProt information and eggNOG-mapper v2.1.7 annotation, concerning entry status, protein name, gene name, and gene ontology identifiers (GO ID).

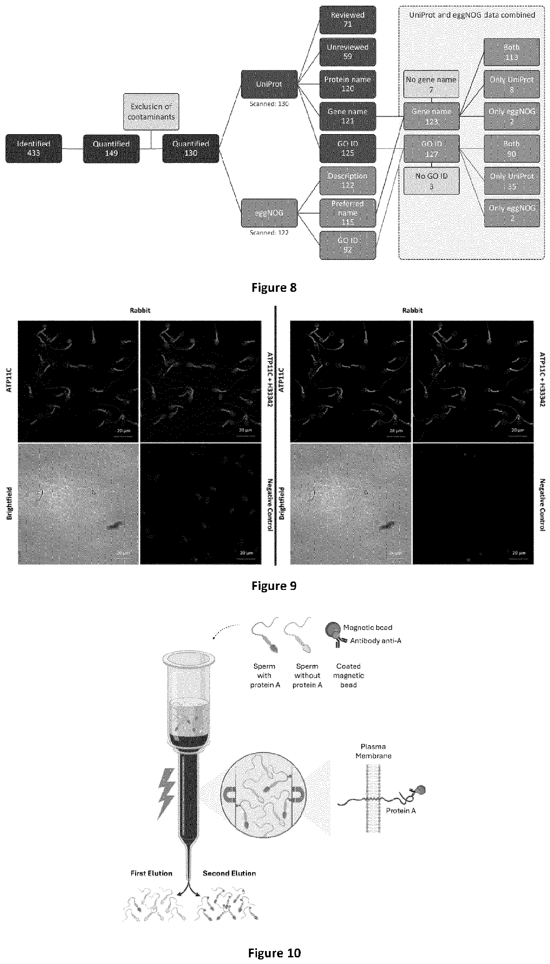

**Figure 9****.** Immunochemistry assay of ATP11C. Differential staining profile of ATP11C in rabbit spermatozoa. In all three rabbit samples tested, 100% of spermatozoa showed staining. However, two distinct staining patterns were observed. In a total of 1311 spermatozoa counted, approximately 49.0±5.6% exhibited staining in the head (primarily in the equatorial segment) and midpiece, while 51.0±5.6% showed staining in the head (also primarily in the equatorial segment), midpiece, and tail. In some spermatozoa, the staining in the equatorial segment was not very intense and, in others, staining was also present in the acrosomal and/or postacrosomal regions.
**Figure 10****.** Illustration depicting the rationale behind the proposed immunological-based sperm sexing technique. When incubating semen samples with magnetic beads coated in antibodies targeting proteins specific to X- or Y-sperm, the respective spermatozoa will bind to the beads. By passing the sample through a magnetic column, the beads will attach to the column walls, enabling the elution of non-bound spermatozoa (first elution). Subsequently, if removing the magnetic field, it will be possible to recover the second population of spermatozoa (second elution).

### Detailed Description of the Disclosure

The present disclosure intends to solve the limitations in sexing technology for mammals, particularly rabbits despite its commercial value, disclosing methods for the identification of protein targets specific of X- and Y-sperm membranes, for sperm sexing technologies.

In one embodiment, aforementioned membrane targets are identified through liquid chromatography with tandem mass spectrometry (LC-MS/MS) to identify potential membrane proteins that could serve as markers to distinguish X- from Y-semen. This approach would thereby contribute to the development of a new sperm sexing technique.

In a further embodiment, bioinformatic analysis of semen samples is performed to identify potential protein candidates for an immunologically based sperm sexing technique.

### EXAMPLES

### Example 1. Bioinformatic determination of potential protein targets for sperm sexing based on the available rabbit X chromosome proteome and human X and Y chromosomes proteome

### 1.1. Materials and methods

To identify potential protein candidates for an immunologically based sperm sexing technique, a bioinformatic analysis was performed (UniProt database, Ensembl BioMart Tool, eggNOG mapper v.2.1.12, DeepTMHMM v.1.0.24, AmiGO 2 GENEONTOLOGY, and two automated scripts were used). To this end, the proteome of the rabbit X chromosome, and the human X and Y chromosomes were analyzed. The analysis was not extended to the Y chromosome, as the Y chromosome proteome is not yet available in the UniProt or NCBI databases. To identify proteins of interest, i.e., those that might be present on the plasma membrane/cell surface, it was considered information about their cellular localization retrieved from the UniProt database and annotations from one-to-one orthology if these had experimental evidence. Nonetheless, to be used as potential targets, the proteins should be accessible from the cell surface and, therefore, preference should be given to cell surface proteins, plasma membrane proteins located at the external side of the membrane or that have a transmembrane domain, or to transmembrane proteins associated with the extracellular space. For this, the analysis was complemented with a software analysis to predict the protein topology and the presence of transmembrane regions in these proteins based on their sequences.

The materials and methods are described in Pinto-Pinho *et al.* (2024) (DOI:10.3390/ani14020217).

### 1.2. Results

### 1.2.1. Proteomes filtering process

A total of 1215 ID entries associated with the rabbit X chromosome were retrieved from UniProt, as well as 2626 entries for the human X chromosome and 91 entries for the human Y chromosome. Following filtering, a total of 676, 834, and 38 UniProt entries were retained for further analysis, respectively (data not shown).

### 1.2.2. Analysis of the sex chromosomes proteome

By integrating UniProt information for each protein entry with eggNOG's functional annotation through fast orthology assignment, a more comprehensive characterization of the selected proteins for further analysis was achieved, particularly in terms of gene names and GO information.

### 1.2.2.1. Characterization of the rabbit X chromosome proteome

By retrieving GO information from UniProt (n=524, 77.5%) and eggNOG (n=307, 45.4%), it was possible to annotate 539 rabbit protein entries (79.7%). Of these, a total of 108 entries (UniProt, n=65; eggNOG, n=87) had GO IDs that could indicate a possible cellular localization of interest. This information was complemented with the topology prediction performed with the deepTMHMM software (data not shown). In total, 519 proteins were predicted to be of the globular type, while 130 were predicted to be al-pha-helical transmembrane proteins, and 27 proteins were exclusively predicted to have a signal peptide. Among the transmembrane proteins, 29 were also predicted to have an SP and the transmembrane regions ranged from 1 to 18.

By combining all available data (data not shown), it was possible to identify a group of 100 proteins of interest that may be present in the plasma membrane, from which 61 are possibly accessible from the cell surface (rabbit X-targets) (Table 1). These proteins represent a group of particular interest for further investigation, due to their potential use as protein targets for sperm sexing. Out of the 61 possible targets, 46 have been selected based on experimentally validated GO information, although not necessarily in spermatozoa.

**Table 1. List of the 61 proteins associated with the rabbit X chromosome that have been identified as potentially accessible from the cell surface through bioinformatic analysis. TM. AI-pha-helical transmembrane protein, SP. signal peptide, SP+TM. Alpha-helical transmembrane protein with signal peptide, GLOB. Globular protein, GO terms. Gene Ontology terms related to the plasma membrane, cell surface, or extracellular space, PM. Plasma Membrane, CS. Cell surface, ESPM. External side of plasma membrane, ACPM. Anchored component of plasma membrane, ACESPM. Anchored component of external side of plasma membrane, ICPM. Integral component of plasma membrane, ES. Extracellular space, *. Preferred name annotated with eggNOG-mapper v.2.1.12 tool (one-to-one orthology, e-value 3.83e-41 to 0.0), as no gene name was available in UniProt, †. Selected based on GO with experimental evidence inferred using eggNOG.**

| **Protein ID** | **Gene name** | **Protein name** | **Topology** | **GO terms** |
|---|---|---|---|---|
| G1TEF4 | *ACE2* | Angiotensin-converting enzyme | SP+TM | CS, PM, ES^{†} |
| G1SSU5 | *ADGRG2* | Adhesion G protein-coupled receptor G2 | SP+TM | PM^{†} |
| Q8MKE9 | *AGTR2* | Type-2 angiotensin II receptor | TM | PM^{†} |
| G1SMQ1 | *AMOT* | Angiomotin | GLOB | CS, PM, ESPM^{†} |
| G1T923 | *ATP6AP2* | Renin receptor | SP+TM | CS, PM, ESPM^{†} |
| G1T6U3 | *ATP7A* | P-type Cu(+) transporter | TM | PM^{†} |
| G1TUV0 | *BR53* | Bombesin receptor subtype-3 | TM | PM^{†} |
| G1SNI0 | *CACNA1F* | Calcium voltage-gated channel subunit alpha1 F | TM | PM^{†} |
| G1SV48 | *CD24** | CD24 molecule | SP | CS, PM, ESPM, ACPM, ACESPM^{†} |
| G1SKP7 | *CD40LG* | CD40 ligand | TM | CS, PM, ESPM, ES^{†} |
| Q9TTU3 | *CLCN5* | H(+)/Cl(-) exchange transporter 5 | TM | PM^{†} |
| G1TSU9 | *CLDN2* | Claudin 2 | TM | PM |
| G1T4N6 | *CLTRN* | Collectrin, amino acid transport regulator | SP+TM | PM^{†} |
| A0A5F9CEX7 | *CXCR3* | C-X-C chemokine receptor type 3 | TM | CS, PM, ESPM^{†} |
| A0A5F9CNI6 | *CYBB* | Cytochrome b-245 beta chain | TM | PM, ICPM^{†} |
| G1TB31 | *CYSLTR1* | Cysteinyl leukotriene receptor 1 | TM | PM, ICPM^{†} |
| G1T9S6 | *EDA* | Ectodysplasin A | TM | PM, ES, ICPM^{†} |
| A0A5F9D0L9 | *EDA2R* | Ectodysplasin A2 receptor | SP+TM | PM |
| A0A5F9CVB5 | *EFNB1* | Ephrin B1 | SP+TM | PM^{†} |
| G1TPG8 | *ENOX2* | Ecto-NOX disulfide-thiol exchanger 2 | GLOB | CS, PM, ESPM^{†} |
| A0A5F9CPD1 | *GDPD2* | Glycerophosphodiester phosphodiesterase domain containing 2 | TM | PM^{†} |
| G1TMB8 | *GJB1* | Gap junction protein | TM | PM^{†} |
| G1T1J4 | *GLRA2* | Glycine receptor alpha 2 | SP+TM | PM, ICPM^{†} |
| G1T836 | *GLRA4** | Glycine receptor alpha 4 | SP+TM | PM, ICPM^{†} |
| A0A5F9DIT5 | *GPC4* | Glypican 4 | TM | CS, PM, ESPM^{†} |
| G1SN36 | *GPR174* | G protein-coupled receptor 174 | TM | PM |
| G1SD16 | *GRIA3* | Glutamate receptor | SP+TM | PM, ICPM^{†} |
| G1T4A8 | *GRPR* | Gastrin releasing peptide receptor | TM | PM, ICPM^{†} |
| G1SXP0 | *GUCY2F* | Guanylate cyclase | SP+TM | PM^{†} |
| A0A5F9C5Z9 | *HEPH* | Hephaestin | SP+TM | PM^{†} |
| A0A5F9DJ94 | *HNRNPM** | RRM domain-containing protein | GLOB | CS^{†} |
| G1TAG7 | *HTR2C* | 5-hydroxytryptamine receptor 2C | SP+TM | CS, PM, ESPM, ICPM^{†} |
| G1TBS6 | *IL1RAPL1* | Interleukin-1 receptor accessory protein-like 1 | SP+TM | CS, PM^{†} |
| G1TPE7 | *IL2RG** | Cytokine receptor common subunit gamma | TM | CS, PM, ESPM^{†} |
| G1SVL1 | *ITM2A* | Integral membrane protein 2 | TM | PM |
| G1TSW5 | *KCNE5* | Potassium voltage-gated channel subfamily E regulatory subunit 5 | TM | PM, ICPM^{†} |
| U3KP42 | *MMGT1** | Membrane magnesium transporter | TM | PM^{†} |
| G1SCP8 | *MSN** | Moesin | GLOB | CS, PM^{†} |
| G1TU56 | *NDP* | Norrin cystine knot growth factor NDP | SP | CS, ES^{†} |
| G1TYL9 | *NLGN3* | Neuroligin 3 | SP+TM | CS, PM, ICPM^{†} |
| G1SPD7 | *NOX1* | NADPH oxidase 1 | TM | PM, ICPM^{†} |
| G1T124 | *OR13H1* | Olfactory receptor family 13 subfamily H member 1 | TM | PM |
| G1TCY0 | *P2RY4* | P2Y purinoceptor 4 | TM | PM^{†} |
| A0A5F9DQS9 | *PCDH11X** | Cadherin domain-containing protein | SP+TM | PM |
| G1TAD7 | *PCDH19* | Protocadherin 19 | SP+TM | PM |
| G1SQ59 | *PHEX* | Phosphate regulating endopeptidase homolog X-linked | TM | CS^{†} |
| G1T949 | *PORCN* | Porcupine O-acyltransferase | TM | PM, ICPM^{†} |
| G1T7A1 | *PTCHD1* | Patched domain containing 1 | TM | PM^{†} |
| G1SZZ7 | *RS1* | Retinoschisin 1 | SP | PM, ESPM, ES |
| G1SZJ1 | *SLC7A3* | Solute carrier family 7 member 3 | TM | PM^{†} |
| G1T4W3 | *SLC9A6* | Sodium/hydrogen exchanger | SP+TM | PM^{†} |
| G1SLC9 | *SLC9A7* | Sodium/hydrogen exchanger | SP+TM | PM |
| G1T0T4 | *SRPX2* | Sushi repeat containing protein X-linked 2 | SP | CS, PM, ES^{†} |
| G1SN13 | *TENM1* | Teneurin transmembrane protein 1 | TM | PM, ICPM^{†} |
| O62852 | *TRPCS* | Short transient receptor potential channel 5 | TM | PM |
| A0A5F9CK03 | *VSIG1* | V-set and immunoglobulin domain-containing protein 1 | SP+TM | PM^{†} |
| G1SR92 | *XK* | XK-related protein | TM | PM |
| A0A5F9CW02 | - | Transmembrane protein 182 | TM | PM |
| G1THX1 | - | Sodium/hydrogen exchanger | TM | PM |
| G1TL60 | - | Olfactory receptor | TM | PM |
| U3KP07 | - | receptor protein-tyrosine kinase | TM | PM |

### 1.2.2.2. Characterization of the human X and Y chromosomes proteome

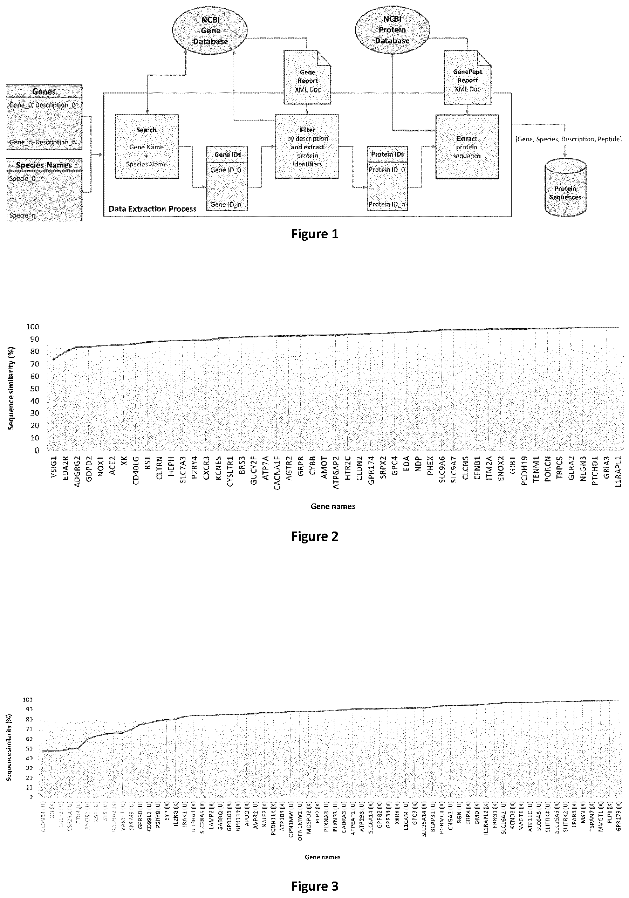

GO information was extracted from UniProt for human X and Y chromosome proteins, resulting in annotations for 740 (X, 88.7%) and 37 (Y, 97.4%) protein entries (Figure 2). Additionally, data was obtained from eggNOG, yielding GO annotations for 492 (X, 59.0%) and 8 (Y, 21.1%) protein entries (data not shown). Therefore, a total of 740 (X, 88.7%) and 37 (Y, 97.4%) protein entries were annotated with GO IDs, respectively. Of these, 231 (X; UniProt, n=212; eggNOG, n=129) and 3 (Y; UniProt, n=2; eggNOG, n=1) had GO IDs indicating a cellular localization of interest. Regarding the topology prediction of the X and Y chromosome proteins, 619 (X) and 35 (Y) were predicted to be globular proteins, 177 (X) and 2 (Y) alpha-helical transmembrane proteins, and 38 (X) and 1 (Y) were exclusively predicted to have a signal peptide. Within the set of transmembrane proteins, 56 (X) and 2 (Y) were further anticipated to have a signal peptide and the transmembrane regions ranged between 1-24 (X) and 1 (Y).

The consolidation of all available data regarding the human X chromosome permitted the identification of a cluster of 211 proteins associated with the plasma membrane, among which 132 are potentially accessible from the cell surface (human X-targets) (Table 2). Out of the 132 possible targets, 75 have been selected based on experimentally validated GO information, although not necessarily in spermatozoa.

**Table 2. List of the 132 proteins associated with the human X chromosome that have been identified as potentially accessible from the cell surface through bioinformatic analysis. TM. Alpha-helical transmembrane protein, SP. signal peptide, SP+TM. Alpha-helical transmembrane protein with signal peptide, GLOB. Globular protein, GO terms. Gene Ontology terms related to the plasma membrane, cell surface, or extracellular space, PM. Plasma Membrane, CS. Cell surface, ESPM. External side of plasma membrane, ACPM. Anchored component of plasma membrane, ICPM. Integral component of plasma membrane, ES. Extracellular space, *. Preferred name annotated with eggNOG-mapper v.2.1.12 tool (one-to-one orthology, e-value 2.11e-232 and 1.55e-59), as no gene name was available in UniProt, †. Selected based on GO with experimental evidence inferred using eggNOG, ††. Selected based on GO with experimental evidence obtained through UniProt and inferred using eggNOG.**

| **Protein ID** | **Gene name** | **Protein name** | **Topolog y** | **GO terms** |
|---|---|---|---|---|
| Q9BYF1 | *ACE2* | Angiotensin-converting enzyme 2 | SP+TM | CS, PM, ES^{††} |
| Q8IZP9 | *ADGRG2* | Adhesion G-protein coupled receptor G2 | SP+TM | CS, PM^{†} |
| P50052 | *AGTR2* | Type-2 angiotensin II receptor | TM | PM^{†} |
| Q99217 | *AMELX* | Amelogenin, X isoform | SP | CS^{†} |
| Q4VCS5 | *AMOT* | Angiomotin | GLOB | CS, PM, ESPM^{†} |
| P23352 | *ANOS1* | Anosmin-1 | SP | CS, PM, ESPM, ES^{†} |
| Q9BUR5 | *APOO* | MICOS complex subunit MIC26 | TM | ES |
| Q8NB49 | *ATP11C* | Phospholipid-transporting ATPase IG | TM | PM^{†} |
| Q9UN42 | *ATP1B4* | Protein ATP1B4 | TM | PM |
| Q16720 | *ATP2B3* | Plasma membrane calcium-transporting ATPase 3 | TM | PM^{†} |
| Q15904 | *ATP6AP1* | V-type proton ATPase subunit S1 | SP+TM | PM^{†} |
| O75787 | *ATP6AP2* | Renin receptor | SP+TM | CS, PM, ESPM^{†} |
| Q04656 | *ATP7A* | Copper-transporting ATPase 1 | TM | PM^{†} |
| P30518 | *AVPR2* | Vasopressin V2 receptor | TM | PM |
| P51572 | *BCAP31* | B-cell receptor-associated protein 31 | TM | PM, ICPM^{†} |
| P21810 | *BGN* | Biglycan | SP | CS, PM, ES^{†} |
| P32247 | *BR53* | Bombesin receptor subtype-3 | TM | PM^{†} |
| O60840 | *CACNA1F* | Voltage-dependent L-type calcium channel subunit alpha-1F | TM | PM^{†} |
| P29965 | *CD40LG* | CD40 ligand | TM | CS, PM, ESPM, ES^{†} |
| P14209 | *CD99* | CD99 antigen | SP+TM | PM |
| Q8TCZ2 | *CD99L2* | CD99 antigen-like protein 2 | SP+TM | CS, PM^{†} |
| P51795 | *CLCN5* | H(+)/Cl(-) exchange transporter 5 | TM | PM^{†} |
| P57739 | *CLDN2* | Claudin-2 | TM | PM |
| H7C241 | *CLDN34* | Claudin-34 | TM | PM |
| Q9HBJ8 | *CLTRN* | Collectrin | SP+TM | PM^{†} |
| A0A3B3IT0 9 | *CLTRN** | Collectrin domain-containing protein | TM | PM |
| Q16280 | *CNGA2* | Cyclic nucleotide-gated olfactory channel | TM | PM |
| Q9HC73 | *CRLF2* | Cytokine receptor-like factor 2 | SP+TM | CS, PM, ESPM^{†} |
| P15509 | *CSF2RA* | Granulocyte-macrophage colony-stimulating factor receptor subunit alpha | SP+TM | PM, ESPM^{†} |
| Q5H943 | *CT83* | Kita-kyushu lung cancer antigen 1 | TM | PM |
| P49682 | *CXCR3* | C-X-C chemokine receptor type 3 | TM | CS, PM, ESPM^{†} |
| P04839 | *CYBB* | Cytochrome b-245 heavy chain | TM | PM, ICPM^{†} |
| Q9Y271 | *CYSLTR1* | Cysteinyl leukotriene receptor 1 | TM | PM, ICPM^{†} |
| P11532 | *DMD* | Dystrophin | GLOB | CS, PM^{†} |
| Q92838 | *EDA* | Ectodysplasin-A | TM | PM, ES, ICPM^{†} |
| Q9HAV5 | *EDA2R* | Tumor necrosis factor receptor superfamily member 27 | TM | PM |
| P98172 | *EFNB1* | Ephrin-B1 | SP+TM | CS, PM^{†} |
| Q16206 | *ENOX2* | Ecto-NOX disulfide-thiol exchanger 2 | GLOB | CS, PM, ESPM, ES^{†} |
| P34903 | *GABRA3* | Gamma-aminobutyric acid receptor subunit alpha-3 | SP+TM | PM |
| Q9UN88 | *GABRQ* | Gamma-aminobutyric acid receptor subunit theta | SP+TM | PM |
| Q9HCC8 | *GDPD2* | Glycerophosphoinositol inositol phosphodiesterase | TM | PM^{†} |
| P08034 | *GJ81* | Gap junction beta-1 protein | TM | PM^{†} |
| P23416 | *GLRA2* | Glycine receptor subunit alpha-2 | SP+TM | PM, ICPM^{†} |
| P51654 | *GPC3* | Glypican-3 | SP | CS, PM, ACPM |
| O75487 | *GPC4* | Glypican-4 | SP | CS, PM, ESPM^{†} |
| Q13491 | *GPM6B* | Neuronal membrane glycoprotein M6-b | TM | PM^{†} |
| Q96P66 | *GPR101* | Probable G-protein coupled receptor 101 | TM | PM |
| Q8TDV5 | *GPR119* | Glucose-dependent insulinotropic receptor | TM | PM |
| P51810 | *GPR143* | G-protein coupled receptor 143 | TM | PM^{†} |
| Q9NS66 | *GPR173* | Probable G-protein coupled receptor 173 | TM | PM |
| Q9BXC1 | *GPR174* | Probable G-protein coupled receptor 174 | TM | PM |
| Q9UPC5 | *GPR34* | Probable G-protein coupled receptor 34 | TM | PM |
| Q13585 | *GPR50* | Melatonin-related receptor | TM | PM^{†} |
| Q96P67 | *GPR82* | Probable G-protein coupled receptor 82 | TM | PM |
| P42263 | *GRIA3* | Glutamate receptor 3 | SP+TM | PM, ICPM^{†} |
| P30550 | *GRPR* | Gastrin-releasing peptide receptor | TM | PM, ICPM^{†} |
| P51841 | *GUCY2F* | Retinal guanylyl cyclase 2 | SP+TM | PM^{†} |
| Q9BQS7 | *HEPH* | Hephaestin | SP+TM | PM^{†} |
| P28335 | *HTR2C* | 5-hydroxytryptamine receptor 2C | SP+TM | CS, PM, ESPM, ICPM^{†} |
| P78552 | *IL13RA1* | Interleukin-13 receptor subunit alpha-1 | SP+TM | PM, ESPM |
| Q14627 | *IL13RA2* | Interleukin-13 receptor subunit alpha-2 | SP+TM | ESPM, ES |
| Q9NZN1 | *IL1RAPL1* | Interleukin-1 receptor accessory protein-like 1 | SP+TM | CS, PM^{†} |
| Q9NP60 | *IL1RAPL2* | X-linked interleukin-1 receptor accessory protein-like 2 | SP+TM | PM |
| P31785 | *IL2RG* | Cytokine receptor common subunit gamma | SP+TM | CS, PM, ESPM^{†} |
| A0A2R8YE7 3 | *IL2RG** | Fibronectin type-III domain-containing protein | SP+TM | CS, PM, ESPM^{†} |
| P26951 | *IL3RA* | Interleukin-3 receptor subunit alpha | SP+TM | PM, ESPM |
| Q01113 | *IL9R* | Interleukin-9 receptor | SP+TM | PM, ESPM, ES |
| P51617 | *IRAK1* | Interleukin-1 receptor-associated kinase 1 | GLOB | CS, PM |
| O43736 | *ITM2A* | Integral membrane protein 2A | TM | PM |
| Q9NSA2 | *KCND1* | Potassium voltage-gated channel subfamily D member 1 | TM | PM |
| Q9UJ90 | *KCNE5* | Potassium voltage-gated channel subfamily E regulatory beta subunit 5 | TM | PM, ICPM^{†} |
| P32004 | *L1CAM* | Neural cell adhesion molecule L1 | SP+TM | CS, PM, ESPM^{†} |
| P13473 | *LAMP2* | Lysosome-associated membrane glycoprotein 2 | SP+TM | PM, ES^{†} |
| Q99677 | *LPAR4* | Lysophosphatidic acid receptor 4 | TM | PM^{†} |
| Q9H0U3 | *MAGT1* | Magnesium transporter protein 1 | SP+TM | PM |
| Q8N4V1 | *MMGT1* | ER membrane protein complex subunit 5 | TM | PM^{†} |
| Q8NHP6 | *MOSPD2* | Motile sperm domain-containing protein 2 | TM | PM, ICPM^{†} |
| P26038 | *MSN* | Moesin | GLOB | CS, PM, ES^{t} |
| O75949 | *NALF2* | NALCN channel auxiliary factor 2 | TM | PM |
| Q00604 | *NDP* | Norrin | SP | CS, ES^{†} |
| Q9NZ94 | *NLGN3* | Neuroligin-3 | SP+TM | CS, PM, ICPM^{†} |
| Q8N0W4 | *NLGN4X* | Neuroligin-4, X-linked | SP+TM | CS, PM, ICPM^{†} |
| Q9Y5S8 | *NOX1* | NADPH oxidase 1 | TM | PM, ICPM^{†} |
| P04000 | *OPN1LW* | Long-wave-sensitive opsin 1 | TM | PM |
| P04001 | *OPN1MW* | Medium-wave-sensitive opsin 1 | TM | PM |
| P0DN77 | *OPN1MW 2* | Medium-wave-sensitive opsin 2 | TM | PM |
| P0DN78 | *OPN1MW 3* | Medium-wave-sensitive opsin 3 | TM | PM |
| Q8NG92 | *OR13H1* | Olfactory receptor 13H1 | TM | PM |
| O00398 | *P2RY10* | Putative P2Y purinoceptor 10 | TM | PM |
| P51582 | *P2RY4* | P2Y purinoceptor 4 | TM | PM^{†} |
| Q86VZ1 | *P2RY8* | P2Y purinoceptor 8 | TM | PM |
| Q9BZA7 | *PCDH11X* | Protocadherin-11 X-linked | SP+TM | PM |
| Q8TAB3 | *PCDH19* | Protocadherin-19 | SP+TM | PM |
| O00264 | *PGRMC1* | Membrane-associated progesterone receptor component 1 | TM | PM |
| P78562 | *PHEX* | Phosphate-regulating neutral endopeptidase PHEX | TM | CS, PM^{†} |
| P60201 | *PLP1* | Myelin proteolipid protein | TM | PM^{†} |
| Q04941 | *PLP2* | Proteolipid protein 2 | TM | PM^{†} |
| P51805 | *PLXNA3* | Plexin-A3 | SP+TM | PM^{†} |
| Q9ULL4 | *PLXNB3* | Plexin-B3 | SP+TM | CS, PM^{†} |
| Q9H237 | *PORCN* | Protein-serine O-palmitoleoyltransferase porcupine | TM | PM, ICPM^{†} |
| O14668 | *PRRG1* | Transmembrane gamma-carboxyglutamic acid protein 1 | TM | PM, ES |
| Q9BZD7 | *PRRG3* | Transmembrane gamma-carboxyglutamic acid protein 3 | TM | ES |
| Q96NR3 | *PTCHD1* | Patched domain-containing protein 1 | TM | PM^{†} |
| O15537 | *RS1* | Retinoschisin | SP | PM, ESPM, ES |
| P36021 | *SLC16A2* | Monocarboxylate transporter 8 | TM | PM, ICPM^{†} |
| O95258 | *SLC25A14* | Brain mitochondrial carrier protein 1 | TM | PM |
| P05141 | *SLC25A5* | ADP/ATP translocase 2 | TM | PM |
| Q8WUX1 | *SLC38A5* | Sodium-coupled neutral amino acid transporter 5 | TM | PM |
| Q9UN76 | *SLC6A14* | Sodium- and chloride-dependent neutral and basic amino acid transporter B(0+) | TM | PM |
| P48029 | *SLC6A8* | Sodium- and chloride-dependent creatine transporter 1 | TM | PM |
| Q8WY07 | *SLC7A3* | Cationic amino acid transporter 3 | TM | PM^{†} |
| Q92581 | *SLC9A6* | Sodium/hydrogen exchanger 6 | SP+TM | PM^{†} |
| Q96T83 | *SLC9A7* | Sodium/hydrogen exchanger 7 | SP+TM | PM |
| Q9H156 | *SLITRK2* | SLIT and NTRK-like protein 2 | SP+TM | PM |
| Q8IW52 | *SLITRK4* | SLIT and NTRK-like protein 4 | SP+TM | PM |
| A6NGZ8 | *SMIM9* | Small integral membrane protein 9 | SP+TM | PM |
| P78539 | *SRPX* | Sushi repeat-containing protein SRPX | SP | CS |
| O60687 | *SRPX2* | Sushi repeat-containing protein SRPX2 | SP | CS, PM, ES^{†} |
| P08842 | STS | Steryl-sulfatase | SP+TM | PM |
| P08247 | *SVP* | Synaptophysin | TM | PM^{†} |
| P51864 | *TDGF1P3* | Putative teratocarcinoma-derived growth factor 3 | SP | CS, PM, ES^{†} |
| Q9UKZ4 | *TENM1* | Teneurin-1 | TM | PM, ICPM^{†} |
| Q9NYK1 | *TLR7* | Toll-like receptor 7 | SP+TM | PM^{†} |
| Q9NR97 | *TLR8* | Toll-like receptor 8 | SP+TM | CS, PM, ESPM^{†} |
| Q9BQJ4 | *TMEM47* | Transmembrane protein 47 | TM | PM^{†} |
| Q9UL62 | *TRPC5* | Short transient receptor potential channel 5 | TM | PM |
| P41732 | *TSPAN7* | Tetraspanin-7 | TM | PM |
| P51809 | *VAMP7* | Vesicle-associated membrane protein 7 | TM | CS, PM^{†} |
| Q86XK7 | *VSIG1* | V-set and immunoglobulin domain-containing protein 1 | SP+TM | PM^{†} |
| P55808 | *XG* | Glycoprotein Xg | SP+TM | PM, ICPM^{†} |
| P51811 | *XK* | Endoplasmic reticulum membrane adapter protein XK | TM | PM |
| Q6PP77 | *XKRX* | XK-related protein 2 | TM | PM |

Regarding the human Y chromosome proteome, 3 proteins were associated with the plasma membrane and all of them are potentially accessible from the cell surface (human Y-targets) (Table 3). One of these 3 possible targets has been selected based on experimentally validated GO information, although not necessarily in spermatozoa.

**Table 3. List of the 3 proteins associated with the human Y chromosome that have been identified as potentially accessible from the cell surface through bioinformatic analysis. SP. signal peptide, SP+TM. Alpha-helical transmembrane protein with signal peptide, GO terms. Gene Ontology terms related to the plasma membrane, cell surface, or extracellular space, PM. Plasma Membrane, CS. Cell surface, ^{†}. Selected based on GO with experimental evidence inferred using eggNOG.**

| **Protein ID** | **Gene name** | **Protein name** | **Topology** | **GO terms** |
|---|---|---|---|---|
| Q99218 | *AMELY* | Amelogenin, Y isoform | SP | CS^{†} |
| Q8NFZ3 | *NLGN4Y* | Neuroligin-4, Y-linked | SP+TM | CS, PM |
| Q9BZA8 | *PCDH11Y* | Protocadherin-11, Y-linked | SP+TM | PM |

### 1.2.3. Cross-species analysis: identification of human targets in the rabbit proteome

A script was used to identify rabbit protein entries with gene and protein names similar to the selected human targets, followed by the determination of the percentage of sequence similarity between the two species. From the 132 human targets, only 130 had a gene and protein name associated according to UniProt and the script was able to identify 118 of them in the rabbit proteome. A total of 49 proteins were common to the list of rabbit X-targets with known gene names, sharing more than 70% of similarity with the human protein sequences (Figure 2). After manual curation, as described in Pinto-Pinho *et al.* (2024) (DOI:10.3390/ani14020217), a list of 66 protein entries were further explored due to their potential to be extra rabbit X-targets - 36 proteins known to be encoded by the rabbit X chromosome and another 30 still unplaced (Figure 3). More than 80% (n=55) of these proteins share more than 70% similarity with the rabbit protein sequences identified. Moreover, by combining all data, a total of 114 possible common entries were identified between humans and rabbits, of which 60 represent potential additional rabbit X-targets determined based on the human X-targets, 33 of them encoded by the rabbit X chromosome and 27 still unplaced in the rabbit proteome. Combining this with the previously defined list of 61 potential rabbit X-targets (Table 1) yields a comprehensive list of 121 potential rabbit X-targets. The 60 proteins added are listed in Table 4.

**Table 4. List of the 60 potential additional rabbit targets of the X chromosome determined based on the human targets of the X chromosome. For each possible additional target, the protein reference sequence identifier of the NCBI database (RefSeq) for the human and rabbit entries, gene name, the protein description, protein sequence similarity between humans and rabbits (%), and the associated rabbit chromosome are described.**

| **RefSeq_Human** | **RefSeq_Rabbit** | **Gene Name** | **Protein description** | **Similarity (%)** | **Chromoso me** |
|---|---|---|---|---|---|
| XP_054183011 | XP_051689653 | *ANOS1* | anosmin-1 | 59.2 | Unplaced |
| XP_054183834 | XP_051683595 | *APOO* | MICOS complex subunit MIC26 | 85.4 | X |
| XP_054182860 | XP_051687539 | *ATP11C* | phospholipid-transporting ATPase IG | 97.3 | Unplaced |
| XP_016884870 | XP_008271374 | *ATP1B4* | protein ATP1B4 | 87.0 | X |
| XP_016885042 | XP_051693419 | *ATP2B3* | plasma membrane calcium-transporting ATPase 3 | 90.5 | Unplaced |
| NP_001174 | NP_001164848 | *ATP6AP1* | V-type proton ATPase subunit S1 precursor | 90.4 | Unplaced |
| NP_000045 | XP_008248538 | *AVPR2* | vasopressin V2 receptor | 86.0 | Unplaced |
| NP_001132929 | XP_008248542 | *BCAP31* | B-cell receptor-associated protein 31 | 92.7 | Unplaced |
| XP_054183538 | XP_051693405 | *BGN* | biglycan | 94.0 | Unplaced |
| XP_047298518 | XP_008273252 | *CD99L2* | CD99 antigen-like protein 2 | 76.5 | Unplaced |
| NP_005131 | XP_051689801 | *CNGA2* | cyclic nucleotide-gated olfactory channel | 94.0 | Unplaced |
| XP_011544483 | XP_008273625 | *CRLF2* | cytokine receptor-like factor 2 | 47.9 | Unplaced |
| XP_047297804 | XP_008249542 | *CSF2RA* | granulocyte-macrophage colony-stimulating factor receptor subunit alpha | 49.6 | Unplaced |
| NP_001017978 | XP_008271135 | *CT83* | kita-kyushu lung cancer antigen 1 | 50.5 | X |
| XP_006724531 | XP_051683632 | *DMD* | dystrophin | 94.6 | X |
| XP_054182733 | XP_051689815 | *GABRA3* | gamma-aminobutyric acid receptor subunit alpha-3 | 89.7 | Unplaced |
| XP_011529486 | XP_002721381 | *GABRQ* | gamma-aminobutyric acid receptor subunit theta | 84.7 | Unplaced |
| XP_054182809 | XP_002720349 | *GPC3* | glypican-3 | 91.3 | X |
| NP_473362 | XP_017205202 | *GPR101* | probable G-protein coupled receptor 101 | 84.8 | X |
| NP_848566 | XP_002720339 | *GPR119* | glucose-dependent insulinotropic receptor | 85.1 | X |
| XP_054183230 | XP_008270867 | *GPR173* | probable G-protein coupled receptor 173 | 100.0 | X |
| XP_005272654 | XP_002719905 | *GPR34* | probable G-protein coupled receptor 34 | 90.9 | X |
| XP_011529518 | XP_008273247 | *GPR50* | melatonin-related receptor | 74.7 | Unplaced |
| XP_047297947 | XP_051682990 | *GPR82* | probable G-protein coupled receptor 82 | 90.8 | X |
| XP_054183006 | XP_008271141 | *IL13RA1* | interleukin-13 receptor subunit alpha-1 | 83.8 | X |
| XP_054183007 | XP_051683230 | *IL13RA2* | interleukin-13 receptor subunit alpha-2 | 65.7 | X |
| XP_011529207 | XP_008271052 | *IL1RAPL2* | X-linked interleukin-1 receptor accessory protein-like 2 | 95.4 | X |
| XP_047298053 | XP_051693453 | *IRAK1* | interleukin-1 receptor-associated kinase 1 | 82.7 | Unplaced |
| NP_004970 | XP_002719951 | *KCND1* | potassium voltage-gated channel subfamily D member 1 | 97.1 | X |
| NP_000416 | XP_051693407 | *L1CAM* | neural cell adhesion molecule L1 | 91.3 | Unplaced |
| NP_001116078 | XP_008271376 | *LAMP2* | lysosome-associated membrane glycoprotein 2 | 84.2 | X |
| XP_016884927 | XP_008271183 | *LPAR4* | lysophosphatidic acid receptor 4 | 98.4 | X |
| NP_001354845 | XP_008271178 | *MAGT1* | magnesium transporter protein 1 | 97.3 | X |
| NP_689794 | XP_051683552 | *MOSPD2* | motile sperm domain-containing protein 2 | 88.0 | X |
| XP_054182806 | XP_051683121 | *NALF2* | NALCN channel auxiliary factor 2 | 86.7 | X |
| NP_000504 | NP_001309193 | *OPN1MW* | medium-wave-sensitive opsin 1 | 87.9 | Unplaced |
| XP_005274486 | XP_002724295 | *P2RY8* | P2Y purinoceptor 8 | 78.9 | Unplaced |
| XP_047297954 | XP_008271184 | *P2RY10* | putative P2Y receptor family member 10 | 87.9 | X |
| NP_006658 | XP_002720306 | *PGRMC1* | membrane-associated progesterone receptor component 1 | 93.8 | X |
| NP_000524 | XP_008271259 | *PLP1* | myelin proteolipid protein | 99.6 | X |
| NP_002659 | NP_001075566 | *PLP2* | proteolipid protein 2 | 88.2 | X |
| XP_047298203 | XP_008248506 | *PLXNA3* | plexin-A3 | 88.5 | Unplaced |
| NP_005384 | XP_017194059 | *PLXNB3* | plexin-B3 | 89.0 | Unplaced |
| NP_001135867 | XP_008270716 | *PRRG1* | transmembrane gamma-carboxyglutamic acid protein 1 | 96.3 | X |
| NP_006508 | XP_051691041 | *SLC16A2* | monocarboxylate transporter 8 | 96.8 | Unplaced |
| XP_011529704 | XP_008271409 | *SLC25A14* | brain mitochondrial carrier protein 1 | 91.6 | X |
| NP_001143 | XP_002720308 | *SLC25A5* | ADP/ATP translocase 2 | 98.3 | X |
| XP_054184094 | XP_051682846 | *SLC38A5* | sodium-coupled neutral amino acid transporter 5 | 83.9 | X |
| NP_009162 | XP_002720288 | *SLC6A14* | sodium- and chloride-dependent neutral and basic amino acid transporter B(0+) | 90.8 | X |
| NP_005620 | NP_001075866 | *SLC6A8* | sodium- and chloride-dependent creatine transporter 1 | 97.5 | Unplaced |
| XP_047298533 | XP_051687602 | *SLITRK2* | SLIT and NTRK-like protein 2 | 98.3 | Unplaced |
| XP_054182427 | XP_008271667 | *SLITRK4* | SLIT and NTRK-like protein 4 | 98.1 | Unplaced |
| NP_001156408 | XP_051691991 | *SMIM9* | small integral membrane protein 9 | 69.7 | Unplaced |
| XP_016885382 | XP_051682971 | *SRPX* | sushi repeat-containing protein SRPX, partial | 94.6 | X |
| XP_047298063 | XP_008247483 | *STS* | steryl-sulfatase | 65.1 | Unplaced |
| NP_003170 | XP_051682874 | *SVP* | synaptophysin | 79.8 | X |
| NP_004606 | XP_017205349 | *TSPAN7* | tetraspanin-7 | 99.2 | X |
| XP_011529490 | XP_002722247 | *VAMP7* | vesicle-associated membrane protein 7 | 66.3 | Unplaced |
| XP_005274644 | XP_008271435 | *XG* | glycoprotein Xg | 47.8 | X |
| XP_011529256 | XP_002720425 | *XKRX* | XK-related protein 2 | 91.2 | X |

### 1.2.4. Cross-reference of the common human and rabbit targets with human spermatozoa proteins

The list of 114 possible common human and rabbit X-targets was cross-referenced with a list of proteins described in human spermatozoa until 23 January 2023, based on a literature search previously conducted by our research group on PubMed, Scopus, and Web of Science databases. By utilizing either the gene name and/or UniProt ID, 53 common proteins were identified, highlighting their potential to be used as targets of X-chromosome-bearing spermatozoa (Table 5). Among these, 24 proteins were originally identified as potential rabbit X-targets in the first analysis (Table 1), while the remaining 29 were part of the additional possible rabbit X-targets identified based on the cross-species analysis (Table 4).

**Table 5. List of the 53 common human and rabbit potential targets of the X chromosome previously described in human spermatozoa. For each target, the UniProt entry ID (Homo sapiens), gene name, and protein name are described. *. Target with gene encoded by the X chromosome in rabbits, t. Target present in the first list of rabbit X-targets obtained (Table 1).**

| **Entry** | **Gene Name** | **Protein Name** |
|---|---|---|
| Q9BYF1 | *ACE2* ^{*†} | Angiotensin-converting enzyme 2 |
| Q8IZP9 | *ADGRG2* ^{*†} | Adhesion G-protein coupled receptor G2 |
| Q4VCS5 | *AMOT* ^{*†} | Angiomotin |
| P23352 | *ANOS1* | Anosmin-1 |
| Q9BUR5 | *APOO** | MICOS complex subunit MIC26 |
| Q8NB49 | *ATP11C* | Phospholipid-transporting ATPase IG |
| Q16720 | *ATP2B3* | Plasma membrane calcium-transporting ATPase 3 |
| Q15904 | *ATP6AP1* | V-type proton ATPase subunit S1 |
| O75787 | *ATP6AP2* ^{*†} | Renin receptor |
| Q04656 | *ATP7A* ^{*†} | Copper-transporting ATPase 1 |
| P51572 | *BCAP31* | B-cell receptor-associated protein 31 |
| P21810 | *BGN* | Biglycan |
| P32247 | *BRS3* ^{*†} | Bombesin receptor subtype-3 |
| O60840 | *CACNA1F* ^{*†} | Voltage-dependent L-type calcium channel subunit alpha-1F |
| P51795 | *CLCN5* ^{*†} | H(+)/Cl(-) exchange transporter 5 |
| P57739 | *CLDN2* ^{*†} | Claudin-2 |
| Q9HC73 | *CRLF2* | Cytokine receptor-like factor 2 |
| Q5H943 | *CT83* ^{*} | Kita-kyushu lung cancer antigen 1 |
| P04839 | *CYBB* ^{*†} | Cytochrome b-245 heavy chain |
| P11532 | *DMD* ^{*} | Dystrophin |
| Q92838 | *EDA* ^{*†} | Ectodysplasin-A |
| Q16206 | *ENOX2* ^{*†} | Ecto-NOX disulfide-thiol exchanger 2 |
| P34903 | *GABRA3* | Gamma-aminobutyric acid receptor subunit alpha-3 |
| Q9HCC8 | *GDPD2* ^{*†} | Glycerophosphoinositol inositolphosphodiesterase GDPD2 |
| P51654 | *GPC3* ^{*} | Glypican-3 |
| O75487 | *GPC4* ^{*†} | Glypican-4 |
| Q14627 | *IL13RA2* ^{*} | Interleukin-13 receptor subunit alpha-2 |
| Q9NZN1 | *IL1RAPL1* ^{*†} | Interleukin-1 receptor accessory protein-like 1 |
| P51617 | *IRAK1* | Interleukin-1 receptor-associated kinase 1 |
| P32004 | *L1CAM* | Neural cell adhesion molecule L1 |
| P13473 | *LAMP2* ^{*} | Lysosome-associated membrane glycoprotein 2 |
| Q9H0U3 | *MAGT1* ^{*} | Magnesium transporter protein 1 |
| Q8N4V1 | *MMGT1* ^{*†} | ER membrane protein complex subunit 5 |
| Q8NHP6 | *MOSPD2* ^{*} | Motile sperm domain-containing protein 2 |
| P26038 | *MSN* ^{*†} | Moesin |
| Q00604 | *NDP* ^{*†} | Norrin |
| Q9Y5S8 | *NOX1* ^{*†} | NADPH oxidase 1 |
| O00264 | *PGRMC1* ^{*} | Membrane-associated progesterone receptor component 1 |
| Q04941 | *PLP2 ^{*}* | Proteolipid protein 2 |
| Q96NR3 | *PTCHD1* ^{*†} | Patched domain-containing protein 1 |
| O95258 | *SLC25A14* ^{*} | Brain mitochondrial carrier protein 1 |
| P05141 | *SLC25A5* ^{*} | ADP/ATP translocase 2 |
| Q9UN76 | *SLC6A14* ^{*} | Sodium- and chloride-dependent neutral and basic amino acid transporter B(0+) |
| Q92581 | *SLC9A6* ^{*†} | Sodium/hydrogen exchanger 6 |
| Q9H156 | *SLITRK2* | SLIT and NTRK-like protein 2 |
| P78539 | *SRPX* ^{*} | Sushi repeat-containing protein SRPX |
| P08842 | STS | Steryl-sulfatase |
| P08247 | *SYP* ^{*} | Synaptophysin |
| Q9UKZ4 | *TENM1* ^{*†} | Teneurin-1 |
| P41732 | *TSPAN7* ^{*} | Tetraspanin-7 |
| P51809 | *VAMP7* | Vesicle-associated membrane protein 7 |
| Q86XK7 | *VSIG1* ^{*†} | V-set and immunoglobulin domain-containing protein 1 |
| P51811 | *XK* ^{*†} | Endoplasmic reticulum membrane adapter protein XK |

### Example 2. Determination of potential protein targets for sperm sexing using rabbit semen samples

To enhance the validation of potential targets, a kit directed for the extraction of proteins from the cell surface was used to extract proteins from rabbit unsexed spermatozoa. Following this, a Liquid Chromatography Tandem Mass Spectrometry (LC-MS/MS) analysis was carried out to analyze the extracted proteins.

### 2.1. Rabbit semen sample acquisition

Three vials of a heterospermic semen pool composed of a mixture of ejaculates from 20 rabbits were provided by Cortagri (Cortegaça Agrícola, Lda, Portugal), according to their validated and implemented procedures. The heterospermic semen pool used in this study presented an average progressive motility of 92.4%, non-progressive motility of 4.1%, and a percentage of immotile spermatozoa of 3.5%. Viable spermatozoa represented 97.0% of the sample. The sample had a sperm concentration of 153.8 × 10⁶ spermatozoa/mL.

### 2.2. Biotinylation and isolation of cell surface proteins

To identify surface proteins of rabbit unsexed spermatozoa (RU), 300 × 10⁶ unsexed spermatozoa were used per technical replicate (n=3). After one wash with room temperature PBS 1X to remove the sperm medium at 450 × g (3 min), the cell surface proteins were biotinylated and isolated with the Pierce^{™} Cell Surface Protein Biotinylation and Isolation Kit (A44390, Thermo ScientificTM), according to slightly modified manufacturer's instructions. The spermatozoa pellets were washed twice with 2 mL of PBS 1X to ensure the removal of the seminal plasma. After biotinylation of the cell surface membrane proteins with EZ-LinkTM Sulfo-NHS-SS-Biotin, the samples were washed twice again with 2 mL of TBS 1X (450 x g, 3 min). Then, the spermatozoa were lysed with the lysis buffer supplemented with protease inhibitors (Halt^{™} Protease Inhibitor Cocktail, 1861278, Thermo Scientific^{™}) during a 60 min incubation on ice. Labeled proteins were then immobilized in NeutrAvidin^{™} Agarose and washed five times with wash buffer, followed by three more washes with 50 mM HEPES (pH 8.0). The labeled proteins were finally eluted with 100 µL of 8 M urea in 50 mM HEPES (pH 8.0) and 100 µL of 20 mM DTT, after an incubation of 45 min, at room temperature, with end-over-end mixing on a rotator. The flowthrough was stored at - 80°C until LC-MS/MS. For the negative control (RC, n=3), the same protocol was followed, but cells were incubated with PBS 1X instead of the biotin solution.

### 2.3. LC-MS/MS

LC-MS/MS was performed at the Flemish Institute for Biotechnology (VIB, Ghent) under the scope of an EPIC-XS project (EPIC-XS-0000186). The samples were sonicated on a Diagenode Bioruptor Plus instrument using the following settings: high-intensity, 10 cycles of 30s ON/30s OFF pulses, +10°C water bath. Proteins were reduced with 15mM DTT for 30min at 55°C and alkylated with 30mM iodoacetamide for 15min at RT in the dark. Samples were diluted twice with 20mM HEPES pH8.0 and proteins were digested with 0.5 µg LysC (1:100, w/w) for 4h at 37°C. Samples were diluted to 2M urea and digested with 0.5 µg trypsin (1:100, w/w) ON at 37°C. The resulting peptide mixture was acidified by the addition of 1% trifluoroacetic acid (TFA) and cleaned up with Phoenix clean-up cartridges (PreOmics) according to the manufacturer's protocol and purified peptides were dried completely in a rotary evaporator. Dried peptides were dissolved in 100µL 50mM triethylammonium bicarbonate (TEAB) and 130IUB mU Peptide-N-Glycosidase F (PNGase F) was added for deglycosylation at 37°C ON. Peptides were acidified with 1% TFA and desalted on reversed-phase (RP) C18 OMIX tips. The tips were first washed 3x with 100µL pre-wash buffer (0.1% TFA in water/acetonitrile (ACN, 20:80, v/v)) and pre-equilibrated 5x with 100µL of wash buffer (0.1% TFA in water) before the sample was loaded on the tip. After peptide binding, the tip was washed 3× with 100µL of wash buffer and peptides were eluted 2× with 100µL elution buffer (0.1% TFA in water/ACN (40:60, v/v)). The combined elutions were transferred to HPLC inserts and dried in a vacuum concentrator.

Peptides were dissolved in 20µL loading solvent A (0.1% TFA in water/ACN (98:2, v/v)) of which 5µL was injected on an Ultimate 3000 RSLCnano system in-line connected to a Q Exactive HF mass spectrometer (Thermo). Trapping was performed at 20µl/min for 2min in loading solvent A on a 5mm trapping column (Thermo scientific, 300µm internal diameter (ID), 5µm beads). The peptides were separated on a 250mm Waters nanoEase M/Z HSS T3 Column, 100Å, 1.8µm, 75µm ID (Waters Corporation) kept at 45°C. Peptides were eluted by a non-linear gradient starting at 1% MS solvent B, reaching 33% MS solvent B (0.1% FA in water/ACN (2:8, v/v)) in 100min, 55% MS solvent B (0.1% FA in water/ACN (2:8, v/v)) in 135min, 70% MS solvent B in 145min followed by a 10-min wash at 70% MS solvent Band re-equilibration with MS solvent A (0.1% FA in water). The mass spectrometer was operated in DDA mode, automatically switching between MS and MS/MS acquisition for the 12 most abundant ion peaks per MS spectrum. Full-scan MS spectra (375-1500 m/z) were acquired at a resolution of 6E4 in the Orbitrap analyzer after accumulation to a target value of 3E6. The 12 most intense ions above a threshold value of 1.5E4 were isolated with a width of 1.5 m/z for fragmentation at a NCE of 30% after filling the trap at a target value of 1E5 for a maximum of 80ms. MS/MS spectra (200-2000 m/z) were acquired at a resolution of 1.5E4 in the Orbitrap analyzer.

Analysis of the mass spectrometry data was performed in MaxQuant (version 2.0.3.0) with mainly default search settings including a false discovery rate set at 1% on PSM, peptide, and protein level. Spectra were searched against the rabbit reference proteome (version of 05 2022, UP000001811 [32]). The mass tolerance for precursor and fragment ions was set to 4.5 and 20 ppm, respectively, during the main search. Enzyme specificity was set as C-terminal to arginine and lysine, also allowing cleavage at proline bonds with a maximum of two missed cleavages. Variable modifications were set to oxidation of methionine residues, acetylation of protein N-termini and deamidation of asparagine and glutamine residues. Matching between runs was enabled with a matching time window of 0.7 min and an alignment time window of 20 min. Only proteins with at least one unique or razor peptide were retained. Proteins were quantified by the MaxLFQ algorithm integrated in the MaxQuant software. A minimum ratio count of two unique or razor peptides was required for quantification. Further data analysis of the shotgun results was performed with an in-house R script, using the protein Groups output table from MaxQuant. Reverse database hits were removed, LFQ intensities were log2 transformed and replicate samples were grouped. Proteins with less than three valid values in at least one group were removed and missing values were imputed from a normal distribution centered around the detection limit (package DEP [45]), leading to a list of 881 quantified proteins in the experiment, used for further data analysis. To compare protein abundance between pairs of sample groups (RU vs RC sample groups), statistical testing for differences between two group means was performed, using the package limma [46]. Statistical significance for differential regulation was set to a false discovery rate (FDR) of <0.05 and |log2FC| = 2. Z-scored LFQ intensities from significantly regulated proteins were plotted in a heatmap after non-supervised hierarchical clustering.

### 2.4. Bioinformatic analysis of the proteome profile of rabbit unsexed spermatozoa

For subsequent analyses, the majority of protein identifiers (IDs) of each quantified protein group were used to avoid accidental hits to a protein group and, from these, only one protein ID per group was considered (unique protein ID). The selected protein ID always corresponded to the first reviewed entry of each protein group or, if none of the entries of the protein group were reviewed, the first one was selected. Functional annotation, transmembrane topology predictions and identification of possible targets was performed as described in Example 1 with minor alterations, namely: proteins associated with plasma membrane-related GO terms, even if not predicted to be transmembrane, were also considered accessible from the cell surface if DeepTMHMM predicted their sequences to be located on the outside of the cell. Moreover, for each identified target, we investigated the presence of orthologues in Homo sapiens (human), Bos taurus (bovine), Ovis aries (sheep), Sus scrofa (pig), and Equus caballus (horse), utilizing the list of orthologues from the NCBI database. The percentage of protein sequence similarity between rabbits and each of these species was determined using the Align tool of the UniProt database. Additionally, to understand which quantified proteins have the potential to be sex-specific targets, the list of quantified proteins obtained by LC-MS/MS was cross-referenced with the list of 121 possible targets previously determined for the rabbit X chromosome (Table 1 and Table 4) and human X (Table 2) and Y chromosomes (Table 3), based on the UniProt ID, and protein and gene names available.

### 2.5. Results

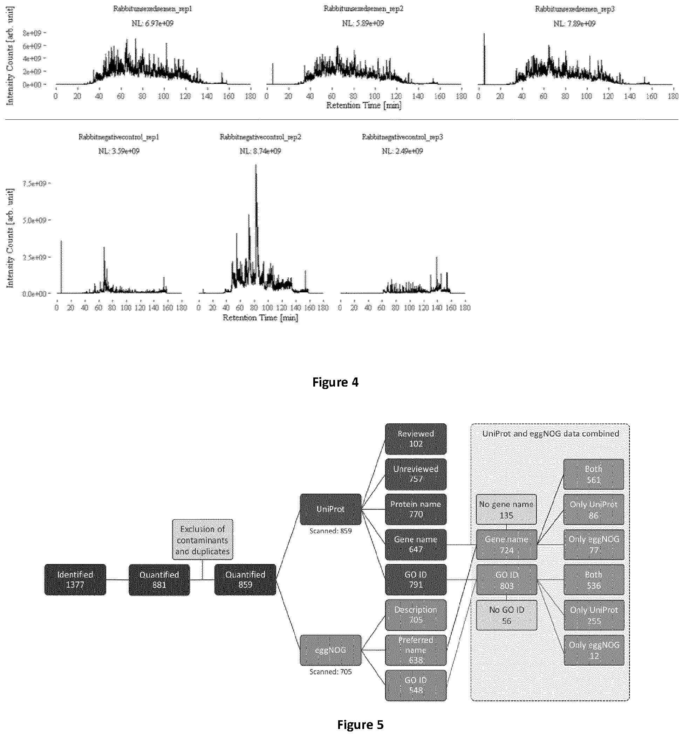

A total of 44,031 peptide-to-spectrum matches (PSMs) were performed, resulting in 6,586 identified unique peptides. Those corresponded to 1,377 identified proteins (data not shown), from which 881 were reliably quantified (data not shown). The LC chromatograms are shown in Figure 4.

After excluding contaminants (corresponding to 17 quantified protein groups) and 5 entries corresponding to unreviewed duplicates of other reviewed entries, a total of 859 protein groups were further analyzed, based on their unique protein IDs (data not shown). All quantified protein groups were present in 2 (n=1) or 3 (n=858) replicates of the unsexed semen samples. The results of the functional annotation are summarized in Figure 5 (data not shown).

By combining the GO information retrieved from UniProt and eggNOG, it was possible to assess in 803 proteins (93%) the existence of GO IDs that could indicate a possible cellular localization in the plasma membrane. This information was complemented with the topology prediction performed with the DeepTMHMM software (data not shown). In total, 574 proteins were predicted to be of the GLOB type, 1 of the BETA type, 151 were predicted to be TM proteins, and 133 proteins were exclusively predicted to have an SP. Among the TM proteins, 62 were also predicted to have an SP, and the TMRs ranged from 1 to 14. By combining all available data (data now shown), it was possible to identify a group of 175 proteins of interest that may be present in the plasma membrane of rabbit spermatozoa, from which 107 are possibly accessible from the cell surface (Table 6). These proteins represent a group of proteins with great potential to be used as protein targets. Those encoded by sex chromosomes may have an even higher potential for sperm sexing applications.

**Table 6. List of the 107 quantified rabbit sperm proteins that have been identified as potentially accessible from the cell surface through bioinformatic analysis. TM. Transmembrane protein, SP. signal peptide, SP+TM. Transmembrane protein with signal peptide, GLOB. Globular protein, GO terms. Gene Ontology terms related to the plasma membrane, cell surface, or extracellular space, PM. Plasma Membrane, CS. Cell surface, ESPM. External side of plasma membrane, ECESPM. Extrinsic component of external side of plasma membrane, ACPM. Anchored component of plasma membrane, ACESPM. Anchored component of external side of plasma membrane, ICPM. Integral component of plasma membrane, ES. Extracellular space.**

| **Protein ID** | **Gene name** | **Protein names** | **Topolog y** | **GO terms** |
|---|---|---|---|---|
| P12822 | *ACE* | Angiotensin-converting enzyme | SP+TM | CS, PM, ESPM, ES, ICPM |
| G1U743 | *ACSL5* | Long-chain-fatty-acid--CoA ligase | TM | PM |
| G1SSU5 | *ADGRG2* | Adhesion G protein-coupled receptor G2 | SP+TM | PM |
| G1SLU6 | *ALPL* | Alkaline phosphatase | GLOB | PM, ES, ACPM |
| A0A5F9DM9 3 | *ANO5* | Anoctamin | TM | PM |
| P15541 | *ANPEP* | Aminopeptidase N | TM | CS, PM, ESPM, ES |
| P51662 | *ANXA1* | Annexin A1 | GLOB | CS, PM, ESPM, ES, ECESPM |
| G1TED6 | *ANXA5* | Annexin | GLOB | CS, PM, ESPM, ES |
| P09809 | *APOA1* | Apolipoprotein A-I | SP | CS, ES |
| G1SUZ7 | *ARSA* | Arylsulfatase A | SP | PM |
| Q9N0Z6 | *ATP1A1* | Sodium/potassium-transporting ATPase subunit alpha-1 | TM | PM, ICPM |
| Q9TT37 | *ATP181* | Sodium/potassium-transporting ATPase subunit beta-1 | TM | PM, ICPM |
| Q9GLC3 | *ATP1B3* | Sodium/potassium-transporting ATPase subunit beta-3 | TM | PM, ICPM |
| P20647 | *ATP2A2* | Sarcoplasmic/endoplasmic reticulum calcium ATPase 2 | TM | PM |
| G1SQA8 | *ATP5F1B* | ATP synthase subunit beta | GLOB | CS, PM |
| G1T923 | *ATP6AP2* | Renin receptor | SP+TM | CS, PM, ESPM |
| G1TER3 | *BCAP31* | B-cell receptor-associated protein | TM | PM, ICPM |
| G1U2S1 | *BSPH1* | Binder of sperm protein homolog 1 | SP | CS |
| G1T7A2 | *C4BPA* | Complement component 4 binding protein alpha | TM | ES |
| P48283 | *CA4* | Carbonic anhydrase 4 | SP | CS, PM, ESPM, ES, ACPM, ACESPM |
| P13806 | *CACNA2D 1* | Voltage-dependent calcium channel subunit alpha-2/delta-1 | SP+TM | PM |
| G1TWP7 | *CACNA2D* 2 | Calcium voltage-gated channel auxiliary subunit alpha2delta 2 | GLOB | PM |
| P15253 | *CALR* | Calreticulin | SP | CS, PM, ESPM, ES |
| G15R15 | *CD109* | CD109 molecule | SP | PM, ES |
| Q28680 | *CD14* | Monocyte differentiation antigen CD14 | SP | CS, PM, ESPM, ES, ACPM, ACESPM |
| O77541 | *CD59* | CD59 glycoprotein | SP | CS, PM, ESPM, ES, ACPM, ACESPM |
| Q9XSC5 | *CLU* | Clusterin | SP | CS, ES |
| A0A5F9DCG4 | *CNTFR* | Ciliary neurotrophic factor receptor | SP | PM, ES, ICPM |
| P31429 | *DPEP1* | Dipeptidase 1 | SP | PM, ES |
| G1SLI2 | *EFNA1* | Ephrin A1 | SP | PM, ACPM |
| G1SVH9 | *ELAPOR1* | KIAA1324 | SP+TM | PM, ICPM |
| G1T7E8 | *ENPP3* | Ectonucleotide pyrophosphatase/phosphodiesterase 3 | TM | CS, PM, ESPM |
| G1T4E0 | *ENPP5* | Ectonucleotide pyrophosphatase/phosphodiesterase family member 5 | SP+TM | PM |
| G1SZ11 | *ENTPD3* | Ectonucleoside triphosphate diphosphohydrolase 3 | TM | PM |
| G1SQK6 | *EPCAM* | Epithelial cell adhesion molecule | SP+TM | CS, PM |
| P04068 | *EPHX1* | Epoxide hydrolase 1 | SP+TM | PM |
| G1SSL0 | *ERP29* | Endoplasmic reticulum resident protein 29 | SP | CS |
| A0A5F9DGH 4 | *ERP44* | Endoplasmic reticulum protein 44 | SP | CS |
| G1TPI0 | *FOLH1* | Folate hydrolase 1 | TM | CS, PM, ICPM |
| A0A5F9C906 | *FUCA1* | Tissue alpha-L-fucosidase | SP | PM |
| A0A5F9CW6 2 | *GPA33* | Glycoprotein A33 | SP+TM | PM, ICPM |
| P43242 | *HMOX2* | Heme oxygenase 2 | TM | PM |
| A0A5F9DF84 | *HNRNPU* | Heterogeneous nuclear ribonucleoprotein U | GLOB | CS |
| A0A5F9CS69 | *HSP90AA1* | Heat shock protein HSP 90-alpha | GLOB | CS, PM |
| P30947 | *HSP90AB1* | Heat shock protein HSP 90-beta | GLOB | CS, PM |
| G1T1V9 | ***HSPA2*** | Heat shock protein family A | GLOB | CS, PM |
| A0A5F9C804 | ***HSPA5*** | 78 kDa glucose-regulated protein | SP | CS, PM, ES |
| G1T3Y8 | *HSPD1* | 60 kDa heat shock protein, mitochondrial | GLOB | CS, PM, ES |
| A0A5F9CYC9 | *IGF2R* | Insulin like growth factor 2 receptor | SP+TM | CS, PM |
| G1TKE7 | *IQGAP2* | IQ motif containing GTPase activating protein 2 | GLOB | CS |
| A0A5F9C4A8 | *IZUMO1* | Izumo sperm-egg fusion 1 | SP+TM | PM |
| G1TRZ2 | *LAMP1* | Lysosomal associated membrane protein 1 | SP+TM | CS, PM, ESPM |
| G1TPZ1 | *LGALS1* | Galectin | GLOB | CS, ES |
| A0A5F9CSC5 | *LIPI* | Lipase I | SP | PM, ES |
| G1SU56 | *LY6G6C* | Lymphocyte antigen 6 family member G6C | SP | ESPM |
| G1U593 | *LY6K* | UPAR/Ly6 domain-containing protein | SP | CS, PM |
| G1T418 | *LY75* | Uncharacterized protein | SP+TM | CS, PM, ESPM |
| G1T521 | *MFGE8* | Uncharacterized protein | GLOB | CS, PM, ESPM, ES |
| P08049 | *MME* | Neprilysin | TM | PM |
| G1TVT0 | *NCL* | Nucleolin | GLOB | CS |
| G1T7J5 | *NCSTN* | Nicastrin | SP+TM | PM, ICPM |
| G1SYH5 | *NPTN* | Neuroplastin | TM | CS, PM |
| U3KNI7 | *NT5E* | 5'-nucleotidase | SP | CS, PM, ESPM |
| A0A5F9CXP2 | *NUCB2* | Nucleobindin 2 | TM | ES |
| P21195 | *P4HB* | Protein disulfide-isomerase | SP | CS, PM, ESPM |
| G1SFR5 | *PAM* | Peptidylglycine alpha-amidating monooxygenase | SP+TM | CS, PM, ES |
| G1U7Z7 | *PCSK5* | Proprotein convertase subtilisin/kexin type 5 | SP+TM | PM, ES |
| G1T0U8 | *PCYOX1* | Prenylcysteine oxidase 1 | SP | PM, ES |
| B7NZF1 | *PDIA3* | Protein disulfide-isomerase | SP | CS, PM, ES |
| G1SFV1 | *PDIA4* | Protein disulfide-isomerase | SP | CS, ES |
| P01832 | *PIGR* | Polymeric immunoglobulin receptor | SP+TM | PM, ES |
| P60990 | *PIP* | Prolactin-inducible protein homolog | SP | PM, ES |
| G1U9Q9 | *PLG* | Plasminogen | SP | ES, ECESPM |
| A0A5F9CXM 9 | *PM20D1* | Peptidase M20 domain containing 1 | TM | ES |
| A0A5F9CHF3 | *PROM1* | Prominin 1 | SP+TM | CS, PM, ES, ICPM |
| G1SMB9 | *PROM2* | Prominin 2 | SP+TM | CS, PM, ICPM |
| G1T384 | *PRSS55* | Serine protease 55 | SP | PM |
| G1SH91 | *PRSS8* | Serine protease 8 | SP | PM, ES, ACPM |
| G1SND0 | *QSOX1* | Sulfhydryl oxidase | SP+TM | ES |
| Q95JD3 | *RHCG* | Ammonium transporter Rh type C | TM | PM, ICPM |
| G1SJL6 | *SCARB2* | Scavenger receptor class B member 2 | TM | PM |
| G1TCC1 | ***SLC16A1*** | Monocarboxylate transporter 1 | TM | PM, ICPM |
| Q9XSC2 | *SLC2A3* | Solute carrier family 2, facilitated glucose transporter member 3 | TM | ICPM |
| G1T377 | *SLC30A1* | Solute carrier family 30 member 1 | TM | PM |
| Q7YQK3 | *SLC3A2* | 4F2 cell-surface antigen heavy chain | TM | CS, PM |
| A0A5F9C2E5 | *SLC44A5* | Choline transporter-like protein | TM | PM |
| G1SJM2 | *SLCO6A1* | Solute carrier organic anion transporter family member | TM | PM |
| G1T661 | *SORT1* | Sortilin 1 | SP+TM | CS, PM |
| P36425 | *SPA17* | Sperm surface protein Sp17 | GLOB | CS, PM, ESPM |
| G1T6C6 | *SPACA4* | Sperm acrosome associated 4 | SP | PM |
| G1U9F1 | *SPAG11B* | Uncharacterized protein | SP | CS, ES |
| G1T1B3 | *SPAM1* | Hyaluronidase | SP | CS, PM, ESPM |
| A0A5F9D6R4 | *STX2* | Syntaxin 2 | TM | CS, PM, ES |
| G1STP9 | *THY1* | Thy-1 membrane glycoprotein | SP | CS, PM, ESPM, ACPM, ACESPM |
| Q28735 | *TMED10* | Transmembrane emp24 domain-containing protein 10 | SP+TM | PM |
| A0A5F9CQ60 | *TMEM30A* | Cell cycle control protein | TM | PM |
| G1SZ75 | *TMEM67* | Transmembrane protein 67 | SP+TM | PM |
| A0A5F9CLK1 | *VAMP3* | Vesicle associated membrane protein 3 | TM | CS, PM |
| A0A5F9DSN1 | *VAPA* | MSP domain-containing protein | TM | PM |
| A0A5F9CK03 | *VSIG1* | V-set and immunoglobulin domain-containing protein 1 | SP+TM | PM |
| P57999 | *ZAN* | Zonadhesin | TM | PM |
| A0A5F9C2X2 | - | Uncharacterized protein | SP+TM | ICPM |
| A0A5F9C6G0 | - | P2X purinoceptor | TM | ICPM |
| A0A5F9DU04 | - | Membrane cofactor protein | SP | CS |
| G15R77 | - | Calcium-transporting ATPase | TM | PM |
| G1TE70 | - | Uncharacterized protein | SP+TM | PM |
| G1TE07 | - | Uncharacterized protein | SP | ACPM |

Following the cross-referencing of the quantified protein list with the rabbit X chromosome proteome, 21 common proteins were identified (Table 7).

**Table 7. List of the 21 quantified rabbit sperm proteins whose genes are encoded by the X chromosome.**

| **Protein ID** | **Gene name** | **Protein name** |
|---|---|---|
| A0A5F9DF97 | *ACOT9* | Acyl-CoA thioesterase 9 |
| G1SSU5 | *ADGRG2* | Adhesion G protein-coupled receptor G2 |
| G1SIM3 | *AIFM1* | Apoptosis inducing factor mitochondria associated 1 |
| G1T923 | *ATP6AP2* | Renin receptor |
| U3KMZ2 | *CYLC1* | Cylicin 1 |
| G1T336 | *DDX3X* | RNA helicase |
| G1U8D5 | *EWSR1* | Uncharacterized protein |
| A0A5F9D270 | *FHL1* | Four and a half LIM domains 1 |
| A0A5F9D1H9 | *GK* | Glycerol kinase |
| G1T044 | *GLA* | Alpha-galactosidase |
| A7X8X3 | *HPRT1* | Hypoxanthine phosphoribosyltransferase |
| G1TES6 | *HSD17B10* | Hydroxysteroid 17-beta dehydrogenase 10 |
| G1SIS9 | *LYZE* | Lysozyme E |
| G1SFF2 | *MAOA* | Amine oxidase |
| G1TT75 | *PGRMC1* | Progesterone receptor membrane component 1 |
| G1TRY5 | *PLS3* | Plastin 3 |
| G1T005 | *PPEF1* | Serine/threonine-protein phosphatase |
| A0A5F9DD95 | *PRDX4* | Peroxiredoxin 4 |
| G1T373 | *PRPS1* | Ribose-phosphate diphosphokinase |
| A0A5F9CK03 | *VSIG1* | V-set and immunoglobulin domain-containing protein 1 |
| G1T7Z6 | - | Phosphoglycerate kinase |

Among these 21 proteins, 6 were of particular interest as they were already associated with the plasma membrane (Table 8), the V-set and immunoglobulin domain-containing protein 1 (VSIG1), the Renin receptor (ATP6AP2), the Adhesion G protein-coupled receptor G2 (ADGRG2), the RNA helicase (DDX3X), the Four and a half LIM domains 1 (FHL1), and the Plastin 3 (PLS3). The first 3 proteins, VSIG1, ATP6AP2, and ADGRG2 are potentially accessible from the cell surface.

**Table 8. List of the 6 quantified rabbit sperm proteins whose genes are encoded by the X chromosome and have been previously associated to the plasma membrane according to the bioinformatic analysis. *Potentially accessible from the cell surface.**

| **Protein ID** | **Gene name** | **Protein name** |
|---|---|---|
| G1SSU5 | ADGRG2 | Adhesion G protein-coupled receptor G2* |
| G1T923 | ATP6AP2 | Renin receptor* |
| G1T336 | DDX3X | RNA helicase |
| A0A5F9D270 | FHL1 | Four and a half LIM domains 1 |
| G1TRY5 | PLS3 | Plastin 3 |
| A0A5F9CK03 | VSIG1 | V-set and immunoglobulin domain-containing protein 1* |

Through a comparative analysis of the quantified proteins with the list of 114 possible common X-targets between rabbits and humans obtained in Example 1, it was found that 5 proteins were common - ATP6AP2, ADGRG2, VSIG1, B-cell receptor-associated protein (BCAP31), and membrane-associated progesterone receptor component 1. These five potential X-targets share orthology among rabbits, humans, cattle, pigs, sheep, and horses. All of them are encoded by the X chromosome in all the mentioned species, except for BCAP31, which, as previously mentioned, is unplaced in rabbits (Table 9).

**Table 9. The 5 potential X-targets identified through the cross-reference of mass spectrometry data from rabbit semen samples with the list of potential common human and rabbit X-targets, with the respective RefSeq identifier and the percentage of similarity between the rabbit protein sequence and the other species. *BCAP and PGRMC1 were selected based on the information available for the human protein.**

| **Species** | | **Proteins** | | | | |
|---|---|---|---|---|---|---|
| | | **ADGRG2** | **ATP6AP2** | **VSIG1** | ***BCAP31** | ***PGRMC1** |
| **Rabbit** | **RefSeq** | XP_0516829 32 | XP_0027198 86 | XP_0082710 80 | XP_0082485 42 | XP_0027203 06 |
| **Human** | **Similarity (%)** | 83.6 | 93.4 | 73.6 | 92.7 | 93.8 |
| | **RefSeq** | XP_0472977 11 | NP_005756 | XP_0052621 84 | NP_0011329 29 | NP_006658 |
| **Bovine** | **Similarity (%)** | 83.3 | 91.4 | 69.7 | 89.4 | 93.3 |
| | **RefSeq** | XP_0248442 47 | NP_0010914 91 | NP_0010393 95 | XP_0052277 49 | NP_0010686 01 |
| **Pig** | **Similarity (%)** | 81.7 | 92 | 71.7 | 87.8 | 95.4 |
| | **RefSeq** | XP_0138414 23 | XP_0031350 70 | XP_0056588 32 | XP_0209353 31 | NP_999076 |
| **Sheep** | **Similarity (%)** | 83.2 | 92.3 | 68.8 | 86.1 | 94.3 |
| | **RefSeq** | XP_0602636 72 | XP_0278185 76 | XP_0040224 82 | XP_0602634 27 | NP_0012955 09 |
| **Horse** | **Similarity (%)** | 86 | 92.9 | 76.6 | 90.2 | 95.4 |
| | **RefSeq** | XP_0056140 86 | NP_0012963 35 | XP_0056144 58 | XP_0056146 82 | XP_0019147 40 |

### Example 3. Determination of potential protein targets for sperm sexing using bovine (un)sexed semen samples

Bovine X-semen (BX), Y-semen (BY), and unsexed semen (BU) are commercially available. Therefore, protein extraction targeting cell surface proteins was performed, followed by LC-MS/MS analysis, to identify potential bovine membrane proteins that could serve as markers to distinguish X-from Y-semen. This approach would thereby contribute to the development of a new sperm sexing technique and complement the analysis performed with the rabbit unsexed semen by revealing potential common targets.

### 3.1. Bovine semen samples acquisition

For the BU condition and negative control (BC), fresh unsexed semen of a 2-year-old Limousin bull was used, provided by Lusogenes (individual motility of 80% and a mass motility score of 4 (on a scale from 1 to 4, as per [47]). For the BX and BY conditions, 2 million X-sorted sperm straws (Holsteins-Frisian) or Y-sorted sperm (Angus and Asturian) were pooled, respectively (purity > 90%; Genex Cooperative and Genoglobal). Both pooled samples met the quality standards set by the commercial suppliers. Moreover, it was assumed that all samples were representative of the population of bulls suitable for breeding in semen collection stations.

### 3.2. Biotinylation, isolation, and LC-MS/MS of cell surface proteins

To identify surface proteins of BX, BY, BU, and BC samples, about 72 × 10⁶ spermatozoa were used per replicate (n=3). Proteins extraction and LC-MS/MS analysis were performed as described previously in Example 2, with minor adaptations. For the bovine samples, 8 µL instead of 5 µL were injected for LC-MS/MS analysis. Also, for data analysis, the bovine reference proteome was used (version 11 2021, UP000009136 [48]). Proteins with less than two valid values in at least one group, instead of three, were removed and missing values were imputed from a normal distribution centered around the detection limit.

### 3.4. Bioinformatic analysis of the proteome profile of bovine (un)sexed spermatozoa

The bioinformatic analysis of the bovine proteome obtained was performed as described in Example 2, with minor adaptations. The UniProt database [34] and the eggNOG-mapper v2.1.7 tool [36] for functional annotation were both accessed on 2022-06-09. The topology of transmembrane proteins was predicted using the DeepTMHMM v.1.0.13 [37]. The bovine quantified proteins were also cross-referenced with the bovine X chromosome proteome and with the human X and Y chromosomes proteome.

For each most promising identified target (transmembrane protein present in 2 or 3 replicas of BX and not in BY), we investigated the presence of orthologues in *Homo sapiens* (human), *Bos taurus* (bovine), *Ovis aries* (sheep), *Sus scrofa* (pig), and *Equus caballus* (horse), utilizing the list of orthologues from the NCBI database, Ensembl BioMart tool, and/or verifying local synteny [49]. The percentage of protein sequence similarity between bovines and each of these species was determined using the Align tool of the UniProt database.

To shed light on possible shared proteins of interest between bovines and rabbits, the quantified proteins in bovines were cross-referenced with those quantified in rabbits, obtained previously in Example 2. A list with all proteins sharing the same gene name was further analyzed to evaluate which of them shared orthology between species. For this, the Ensembl Biomart tool was used, combined with the information available in the NCBI database in terms of orthologs and local synteny for each gene [49]. Among the established ortholog proteins, emphasis was placed on those meeting two conditions: being identified in BX samples but not in BY samples and being considered possibly accessible from the cell surface in both species. Moreover, the list of bovine quantified proteins was cross-checked with the total list of 121 potential rabbit X-targets obtained by bioinformatic analysis in Example 1, based on the available gene name. For proteins identified with a common encoding gene, shared orthology was verified, as described above.

### 3.5. Results

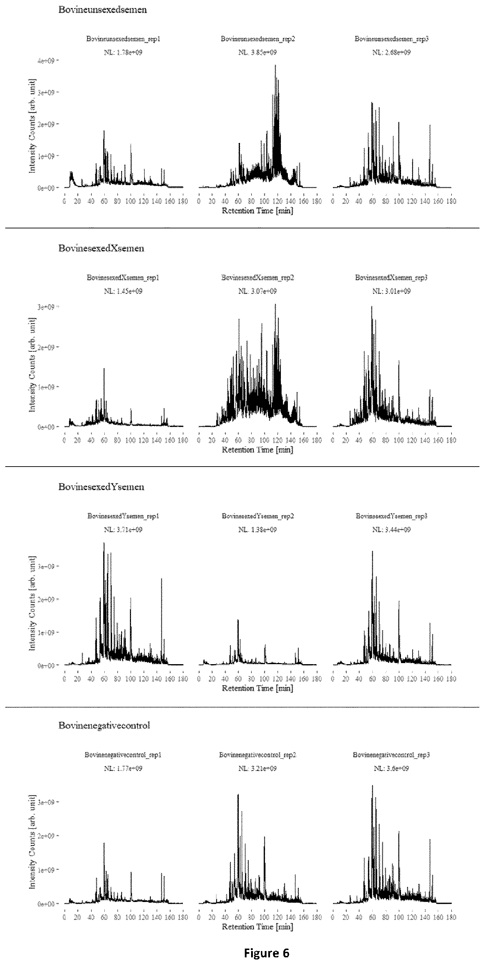

A total of 17,673 peptide-to-spectrum matches (PSMs) were performed, resulting in 1,337 identified unique peptides. These corresponded to 433 identified proteins (data not shown), of which 149 were reliably quantified (data not shown). The LC chromatograms are shown in Figure 6.

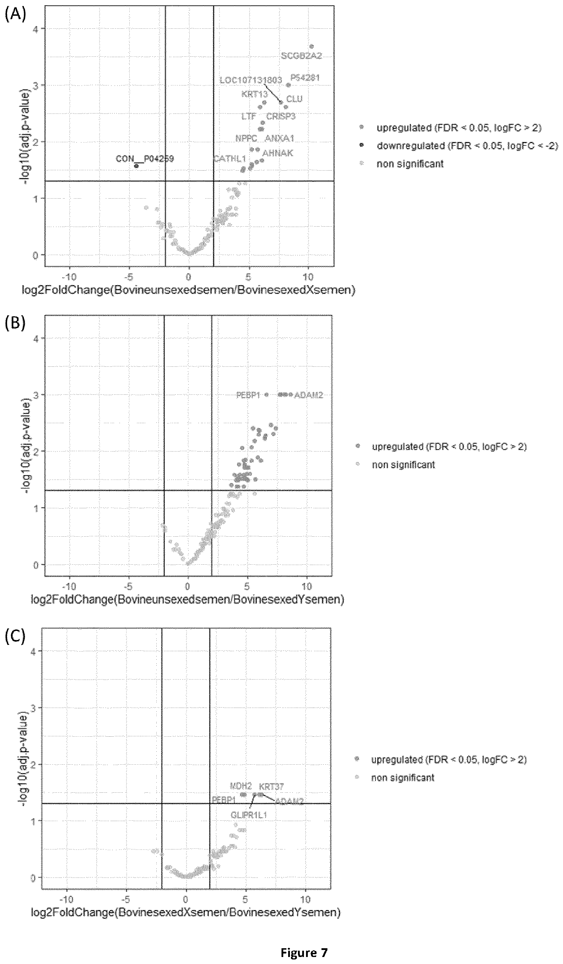

The differential expression testing results (FE < 0.05 and |log2FC| = 2) are shown in Tables 10 (BU vs BX), 11 (BU vs BY), and 12 (BX vs BY). According to this analysis, BU samples had 18 proteins significantly upregulated and 1 protein (contaminant) significantly downregulated when comparing with BX samples (Figure 7A, Table 10), and 52 proteins significantly upregulated when comparing with BY samples (Figure 7B, Table 11).

**Table 10. List of the 19 proteins differentially expressed between bovine unsexed samples (BU) and bovine X-semen samples (BX) (FE < 0.05 and |log2FC| = 2): 18 upregulated and 1 downregulated. FC. Fold Change.**

| **Protein ID** | **Gene name** | **Protein name** | **logFC BU vs BX** | **Adjusted *p-value* BU vs BX** | **Regulation** |
|---|---|---|---|---|---|
| A6QPK0 | *SCGB2A2* | SCGB2A2 protein | 10.23 | 0.0002 | Upregulated |
| P54281 | - | Calcium-activated chloride channel regulator 1 | 8.28 | 0.0010 | Upregulated |
| P17697 | *CLU* | Clusterin | 8.07 | 0.0024 | Upregulated |
| A0A3Q1LMW 6 | *LOC10713180 3* | SERPIN domain-containing protein | 7.64 | 0.0020 | Upregulated |
| A0A3Q1LJB2 | *KRT13* | IF rod domain-containing protein | 6.3 | 0.0020 | Upregulated |
| Q3ZCL0 | *CRISP3* | Cysteine-rich secretory protein 2 | 6.11 | 0.0046 | Upregulated |
| P46193 | *ANXA1* | Annexin A1 | 6.07 | 0.0059 | Upregulated |
| E1B9P4 | *ELSPBP1* | Epididymal sperm binding protein 1 | 6.07 | 0.0215 | Upregulated |
| P55206 | *NPPC* | C-type natriuretic peptide | 5.95 | 0.0059 | Upregulated |
| P24627 | *LTF* | Lactotransferrin | 5.93 | 0.0024 | Upregulated |
| A0A3Q1MHT0 | *AHNAK* | AHNAK nucleoprotein | 5.74 | 0.0136 | Upregulated |
| A0A3Q1LK79 | - | Uncharacterized protein | 5.65 | 0.0231 | Upregulated |
| F1N1Z8 | *LOC10497583 0* | Uncharacterized protein | 5.26 | 0.0255 | Upregulated |
| P20811 | *CAST* | Calpastatin | 5.25 | 0.0268 | Upregulated |
| P22226 | *CATHL1* | Cathelicidin-1 | 5.23 | 0.0136 | Upregulated |
| F1MMY0 | *PTCHD3* | SSD domain-containing protein | 5.09 | 0.0298 | Upregulated |
| P05307 | *P4HB* | Protein disulfide-isomerase | 4.56 | 0.0298 | Upregulated |
| F1MFA3 | *PITRM1* | Presequence protease, mitochondrial | 4.45 | 0.0325 | Upregulated |
| CON__P04259 | - | - | -4.44 | 0.0268 | Downregulate d |

**Table 11. List of the 52 proteins differentially expressed (upregulated) between bovine unsexed samples (BU) and bovine Y-semen samples (BY) (FE < 0.05 and |log2FC| = 2). FC, Fold Change.**

| **Protein ID** | **Gene name** | **Protein name** | **logFC BU vs BY** | **Adjusted *p-value* BU vs BY** | **Regulation** |
|---|---|---|---|---|---|
| A6QPK0 | *SCGB2A2* | SCGB2A2 protein | 8.61 | 0.00098 | Upregulated |
| O77780 | *ADAM2* | Disintegrin and metalloproteinase domain-containing protein 2 | 8.23 | 0.00098 | Upregulated |
| P54281 | - | Calcium-activated chloride channel regulator 1 | 8.12 | 0.00098 | Upregulated |
| A0A3Q1LM W6 | *LOC10713180 3* | SERPIN domain-containing protein | 7.87 | 0.00098 | Upregulated |
| Q32LB5 | *GLIPR1L1* | GLIPR1-like protein 1 | 7.74 | 0.00098 | Upregulated |
| P17697 | *CLU* | Clusterin | 7.40 | 0.00393 | Upregulated |
| E1B9P4 | *ELSPBP1* | Epididymal sperm binding protein 1 | 7.18 | 0.00492 | Upregulated |
| A0A3Q1MH T0 | *AHNAK* | AHNAK nucleoprotein | 6.96 | 0.00348 | Upregulated |
| P13696 | *PEBP1* | Phosphatidylethanolamine-binding protein 1 | 6.58 | 0.00098 | Upregulated |
| E1BG77 | *ADAM32* | ADAM metallopeptidase domain 32 | 6.54 | 0.00521 | Upregulated |
| O77797 | *AKAP3* | A-kinase anchor protein 3 | 6.48 | 0.00563 | Upregulated |
| A0A3Q1LK7 9 | *Adamlb* | Uncharacterized protein | 6.47 | 0.00590 | Upregulated |
| Q0VC36 | *SFN* | 14-3-3 protein sigma | 6.12 | 0.01466 | Upregulated |
| P55206 | *NPPC* | C-type natriuretic peptide | 5.97 | 0.00431 | Upregulated |
| Q3ZCL0 | *CRISP3* | Cysteine-rich secretory protein 2 | 5.94 | 0.00424 | Upregulated |
| A0A3Q1LJ5 9 | *Abca14* | Uncharacterized protein | 5.92 | 0.00503 | Upregulated |
| A0A3Q1M7 X0 | *PRSS44* | Peptidase S1 domain-containing protein | 5.90 | 0.01288 | Upregulated |
| Q3T067 | *SCCPDH* | Saccharopine dehydrogenase-like oxidoreductase | 5.70 | 0.03112 | Upregulated |
| P46193 | *ANXA1* | Annexin A1 | 5.59 | 0.00668 | Upregulated |
| A0A3Q1LJB 2 | *KRT13* | IF rod domain-containing protein | 5.47 | 0.00393 | Upregulated |
| F1MMY0 | *PTCHD3* | SSD domain-containing protein | 5.36 | 0.01466 | Upregulated |
| P05307 | *P4HB* | Protein disulfide-isomerase | 5.33 | 0.00861 | Upregulated |
| P34933 | *HSPA2* | Heat shock-related 70 kDa protein 2 | 5.23 | 0.02550 | Upregulated |
| P20811 | *CAST* | Calpastatin | 5.06 | 0.01945 | Upregulated |
| E1B7S8 | *ACRBP* | Acrosin-binding protein | 5.01 | 0.03275 | Upregulated |
| A0A3Q1NJ7 0 | *PFKP* | ATP-dependent 6-phosphofructokinase | 4.97 | 0.01945 | Upregulated |
| P31081 | *HSPD1* | 60 kDa heat shock protein, mitochondrial | 4.97 | 0.03118 | Upregulated |
| Q9XSJ4 | *ENO1* | Alpha-enolase | 4.96 | 0.02550 | Upregulated |
| P48818 | *ACADVL* | Very long-chain specific acyl-CoA dehydrogenase, mitochondrial | 4.89 | 0.03118 | Upregulated |
| P22226 | *CATHL1* | Cathelicidin-1 | 4.81 | 0.01399 | Upregulated |
| G5E5C6 | *ACR* | Acrosin | 4.76 | 0.03047 | Upregulated |
| P0CB32 | *HSPA1L* | Heat shock 70 kDa protein 1-like | 4.74 | 0.01770 | Upregulated |
| A0A3Q1LUH 6 | *MSMB* | Beta-microseminoprotein | 4.74 | 0.02888 | Upregulated |
| Q04467 | *IDH2* | Isocitrate dehydrogenase [NADP], mitochondrial | 4.73 | 0.01715 | Upregulated |
| Q27975 | *HSPA1A* | Heat shock 70 kDa protein 1A | 4.73 | 0.01945 | Upregulated |
| P19483 | *ATP5F1A* | ATP synthase subunit alpha, mitochondrial | 4.71 | 0.04276 | Upregulated |
| G5E622 | | Uncharacterized protein | 4.68 | 0.01466 | Upregulated |
| F1N1Z8 | *LOC10497583* 0 | Uncharacterized protein | 4.66 | 0.02604 | Upregulated |
| P24627 | *LTF* | Lactotransferrin | 4.56 | 0.00883 | Upregulated |
| P19660 | *CATHL2* | Cathelicidin-2 | 4.53 | 0.0308 | Upregulated |
| A0A3Q1N18 1 | *SCEL* | Sciellin | 4.38 | 0.02604 | Upregulated |
| Q32LG3 | *MDH2* | Malate dehydrogenase, mitochondrial | 4.32 | 0.01715 | Upregulated |
| E1BCR1 | *ABCA3* | ATP binding cassette subfamily A member 3 | 4.32 | 0.03047 | Upregulated |
| Q2YDD9 | *SLC25A31* | ADP/ATP translocase 4 | 4.28 | 0.03118 | Upregulated |
| P61603 | *HSPE1* | 10 kDa heat shock protein, mitochondrial | 4.24 | 0.03270 | Upregulated |
| F1MBQ1 | *PPEF1* | Serine/threonine-protein phosphatase with EF-hands | 4.19 | 0.04276 | Upregulated |
| F1N3G6 | *ATP11C* | Phospholipid-transporting ATPase | 4.16 | 0.03118 | Upregulated |
| F1MFA3 | *PITRM1* | Presequence protease, mitochondrial | 4.15 | 0.02781 | Upregulated |
| 018956 | *ENTPD1* | Ectonucleoside triphosphate diphosphohydrolase 1 | 4.10 | 0.04244 | Upregulated |
| Q2T9N0 | *FSCB* | Fibrous sheath CABYR-binding protein | 4.06 | 0.03308 | Upregulated |
| Q32L61 | *CABVR* | Calcium binding tyrosine phosphorylation regulated | 3.92 | 0.02630 | Upregulated |
| A0A3Q1M7 W7 | *LOC10497019 3* | Uncharacterized protein | 3.62 | 0.03898 | Upregulated |

Regarding possible differences between the X- and Y-sperm proteome, it was found that 5 proteins were significantly upregulated in BX samples (Figure 7C, Table 12) - apart from a contaminant, Keratin 37 (KRT37), these proteins are the GLIPR1-like protein 1 (GLIPR1L1), Phosphatidylethanolamine-binding protein 1 (PEBP1), Disintegrin and metalloproteinase domain-containing protein 2 (ADAM 2), and Malate dehydrogenase, mitochondrial (MDH2).

**Table 12. List of the 5 proteins differentially expressed (upregulated) between bovine X-semen samples (BX) and bovine Y-semen samples (BY) (FE < 0.05 and |log2FC| = 2). FC, Fold Change.**

| **Protein ID** | **Gene name** | **Protein name** | **logFC BX vs BY** | **Adjusted *p-value* BX vs BY** | **Regulation** |
|---|---|---|---|---|---|
| O77780 | *ADAM2* | Disintegrin and metalloproteinase domain-containing protein 2 | 6.35 | 0.035 | Upregulated |
| CON_P02 533 | *KRT37* | Keratin 37 | 6.10 | 0.035 | Upregulated |
| Q32LB5 | *GLIPR1L1* | GLIPR1-like protein 1 | 5.74 | 0.035 | Upregulated |
| Q32LG3 | *MDH2* | Malate dehydrogenase, mitochondrial | 4.86 | 0.035 | Upregulated |
| P13696 | *PEBP1* | Phosphatidylethanolamine-binding protein 1 | 4.68 | 0.035 | Upregulated |

After excluding contaminants (corresponding to 19 quantified protein groups), a total of 130 quantified protein groups were further analyzed, based on their unique protein IDs. A total of 108 quantified proteins were identified in BU samples, whereas in BX and BY samples, 94 and 30 quantified proteins were identified, respectively. To explore further differences in the presence of some proteins between the samples BU, BX, and BY, a non-statistical observation was performed. Atotal of 47 quantified proteins were identified in at least 1 replica in both BU and BX samples, and 17 of the quantified proteins were identified only in BX samples. Therefore, a total of 64 quantified proteins (Table 13) were identified in BX samples and not in BY samples. On the other hand, every quantified protein identified in BY samples was also identified in BX samples, so none of the proteins were identified exclusively in BY samples. Also, no reliably quantified proteins were identified in the BC replicates (data not shown).

**Table 13. List of the 64 quantified bovine sperm proteins that have been identified in bovine X-semen samples (BX) but not in bovine Y-semen samples (BY). TM. Transmembrane protein, SP. signal peptide, SP+TM. Transmembrane protein with signal peptide, GLOB. Globular protein, GO terms. Gene Ontology terms related to the plasma membrane, cell surface, or extracellular space, PM. Plasma Membrane, CS. Cell surface, ESPM. External side of plasma membrane, ACPM. Anchored component of plasma membrane, ICPM. Integral component of plasma membrane, ES. Extracellular space, No. BX replicates. number of BX replicates in which the protein was identified.**

| **Protein ID** | **Gene Name** | **Protein Name** | **Topology** | **GO terms** | **No. BX Replicates** |
|---|---|---|---|---|---|
| O77780 | *ADAM2* | Disintegrin and metalloproteinase domain-containing protein 2 | SP+TM | CS | 3 |
| Q9XSJ4 | *ENO1* | Alpha-enolase | GLOB | PM | 3 |
| Q8MJN0 | *FUNDC2* | FUN14 domain-containing protein 2 | TM | | 3 |
| Q32LB5 | *GLIPR1L1* | GLIPR1-like protein 1 | SP | PM, ES | 3 |
| P31081 | *HSPD1* | Chaperonin 60 | GLOB | CS, PM, ES | 3 |
| Q04467 | *IDH2* | Isocitrate dehydrogenase [NADP], mitochondrial | GLOB | | 3 |
| A0A3Q1MGG8 | *IQCN* | Uncharacterized protein | GLOB | | 3 |
| Q32LG3 | *MDH2* | Malate dehydrogenase, mitochondrial | GLOB | | 3 |
| P13696 | *PEBP1* | Phosphatidylethanolamine-binding protein 1 | GLOB | | 3 |
| A0A3Q1NJ70 | *PFKP* | ATP-dependent 6-phosphofructokinase | GLOB | | 3 |
| A0A3Q1M7X0 | *PRSS44* | Peptidase S1 domain-containing protein | SP | | 3 |
| Q3MHW6 | *SLC16A1* | Monocarboxylate transporter 1 | TM | PM, ICPM | 3 |
| P48818 | *ACADVL* | Very long-chain specific acyl-CoA dehydrogenase, mitochondrial | GLOB | | 2 |
| G5E5C6 | *ACR* | Acrosin | SP | | 2 |
| E1B7S8 | *ACRBP* | Acrosin-binding protein | SP | | 2 |
| A0A3Q1MGC0 | *ACSS1* | Propionate--CoA ligase | GLOB | | 2 |
| A0A3Q1LK79 | *Adamlb* | Uncharacterized protein | SP | PM | 2 |
| E1BG77 | *ADAM32* | ADAM metallopeptidase domain 32 | SP+TM | | 2 |
| Q2KJ64 | *ARG1* | Arginase-1 | GLOB | ES | 2 |
| F1N3G6 | *ATP11C* | Phospholipid-transporting ATPase | TM | PM | 2 |
| D3K0R6 | *ATP2B4* | Plasma membrane calcium-transporting ATPase 4 | TM | ICPM | 2 |
| P00829 | *ATP5F1B* | ATP synthase F1 subunit beta | GLOB | CS, PM | 2 |
| Q5E9F7 | *CFL1* | Cofilin-1 | GLOB | PM | 2 |
| F1N206 | *DLD* | Dihydrolipoyl dehydrogenase | GLOB | | 2 |
| Q03763 | *DSG1* | Desmoglein-1 | SP+TM | PM | 2 |
| P55052 | *FABP5* | Fatty acid-binding protein 5 | GLOB | ES | 2 |
| Q2KJE5 | *GAPDHS* | Glyceraldehyde-3-phosphate dehydrogenase, testis-specific | GLOB | | 2 |
| F1N7X7 | *GARIN3* | Family with sequence similarity 71 member B | GLOB | | 2 |
| Q27975 | *HSPA1A* | Heat shock 70 kDa protein 1A | GLOB | PM | 2 |
| P0CB32 | *HSPA1L* | Heat shock 70 kDa protein 1-like | GLOB | PM | 2 |
| P34933 | *HSPA2* | Heat shock-related 70 kDa protein 2 | GLOB | CS, PM | 2 |
| Q0VCX2 | *HSPA5* | HSP70 family protein 5 | SP | CS, PM, ES | 2 |
| P19120 | *HSPA8* | Heat shock cognate 71 kDa protein | GLOB | PM, ES | 2 |
| Q3ZCH0 | *HSPA9* | Stress-70 protein, mitochondrial | GLOB | | 2 |
| Q2TBQ6 | *HSPB9* | Heat shock protein beta-9 | GLOB | | 2 |
| P61603 | *HSPE1* | 10 kDa heat shock protein, mitochondrial | GLOB | | 2 |
| F1MFW9 | *KRT24* | Keratin 24 | GLOB | | 2 |
| P05786 | *KRTS* | Keratin, type II cytoskeletal 8 | GLOB | PM | 2 |
| F1ML59 | *NT5C1B* | 5'-nucleotidase, cytosolic IB | GLOB | | 2 |
| Q3ZCF5 | *OAT* | Ornithine aminotransferase, mitochondrial | GLOB | | 2 |
| F1MFA3 | *PITRM1* | Presequence protease, mitochondrial | GLOB | | 2 |
| A5D984 | *PKM* | Pyruvate kinase | GLOB | | 2 |
| Q28161 | *PKP1* | Plakophilin-1 | GLOB | PM | 2 |
| F1MBQ1 | *PPEF1* | Serine/threonine-protein phosphatase with EF-hands | GLOB | | 2 |
| P61287 | *PPP1CC* | Serine/threonine-protein phosphatase PP1-gamma catalytic subunit | GLOB | | 2 |
| Q5E947 | *PRDX1* | Peroxiredoxin-1 | GLOB | | 2 |
| A0A3Q1MHX8 | *RAB2A* | RAB2A, member RAS onco family | GLOB | | 2 |
| Q0VC36 | *SFN* | 14-3-3 protein sigma | GLOB | | 2 |
| Q32PB3 | *SPACA4* | Sperm acrosome membrane-associated protein 4 | SP | PM | 2 |
| F1MTV1 | *SPAM1* | Hyaluronidase | SP | PM | 2 |
| F1MKF8 | *SQOR* | Sulfide quinone oxidoreductase | GLOB | | 2 |
| F1MBB7 | *TGM1* | Transglutaminase 1 | TM | | 2 |
| Q3MHM5 | *TUBB4B* | Tubulin beta-4B chain | GLOB | | 2 |
| F1N530 | | Uncharacterized protein | GLOB | | 2 |
| A0A3Q1MHT0 | *AHNAK* | AHNAK nucleoprotein | GLOB | PM, ES | 1 |
| O77797 | *AKAP3* | A-kinase anchor protein 3 | GLOB | | 1 |
| P09487 | *ALPL* | Alkaline phosphatase, tissue-nonspecific isozyme | SP | PM, ES, ACPM | 1 |
| Q3T0C6 | *ATP1B3* | Sodium/potassium-transporting ATPase subunit beta-3 | TM | PM, ICPM | 1 |
| P19483 | *ATP5F1A* | ATP synthase subunit alpha, mitochondrial | GLOB | PM | 1 |
| P62157 | *CALM2* | Calmodulin | GLOB | PM, ICPM | 1 |
| Q2T9N0 | *FSCB* | Fibrous sheath CABYR-binding protein | GLOB | | 1 |
| Q3T056 | *LDHAL6B* | L-lactate dehydrogenase A-like 6B | GLOB | | 1 |
| Q3T067 | *SCCPDH* | Saccharopine dehydrogenase-like oxidoreductase | GLOB | | 1 |
| A0A3Q1MQE3 | - | Uncharacterized protein | SP+TM | PM, ESPM | 1 |

The results of the functional annotation of the quantified proteins are summarized in Figure 8 (data not shown).

By combining the GO information retrieved from UniProt and eggNOG, it was possible to assess in 127 proteins (98%) the existence of GO IDs that could indicate a possible cellular localization in the plasma membrane. This information was complemented with the topology prediction performed with the DeepTMHMM software (data not shown). In total, 81 proteins were predicted to be of the GLOB type, 23 were predicted to be TM proteins, and 26 proteins were exclusively predicted to have an SP. Among the TM proteins, 9 were also predicted to have an SP, and the TMRs ranged from 1 to 12. By combining all data (not shown), it was possible to identify a group of 52 proteins of interest that may be present in the plasma membrane of bovine spermatozoa, from which 39 are possibly accessible from the cell surface.

**Table 14. List of the 39 quantified rabbit sperm proteins that have been identified as potentially accessible from the cell surface through bioinformatic analysis. TM. Transmembrane protein, SP. signal peptide, SP+TM. Transmembrane protein with signal peptide, GLOB. Globular protein, GO terms. Gene Ontology terms related to the plasma membrane, cell surface, or extracellular space, PM. Plasma Membrane, CS. Cell surface, ESPM. External side of plasma membrane, ECESPM. Extrinsic component of external side of plasma membrane, ACPM. Anchored component of plasma membrane, ICPM. Integral component of plasma membrane, ES. Extracellular space.**

| **Protein ID** | **Gene name** | **Protein name** | **Topology** | **GO terms** |
|---|---|---|---|---|
| **E1BCR1** | *ABCA3* | ATP binding cassette subfamily A member 3 | TM | PM |
| **F1MQJ0** | *ACE* | Angiotensin-converting enzyme | SP+TM | CS, PM, ESPM, ES |
| **AOA3Q1LK79** | *Adamlb* | Uncharacterized protein | SP | PM |
| **O77780** | *ADAM2* | Disintegrin and metalloproteinase domain-containing protein 2 | SP+TM | CS |
| **P09487** | *ALPL* | Alkaline phosphatase, tissue-nonspecific isozyme | SP | PM, ES, ACPM |
| **P46193** | *ANXA1* | Annexin A1 | GLOB | CS, PM, ESPM, ES, ECESPM |
| **P04272** | *ANXA2* | Annexin A2 | GLOB | CS, PM, ES |
| **F1N3G6** | *ATP11C* | Phospholipid-transporting ATPase | TM | PM |
| **E1B8N5** | *ATP1A4* | Sodium/potassium-transporting ATPase subunit alpha | TM | PM |
| **Q3T0C6** | *ATP1B3* | Sodium/potassium-transporting ATPase subunit beta-3 | TM | PM, ICPM |
| **D3K0R6** | *ATP2B4* | Plasma membrane calcium-transporting ATPase 4 | TM | ICPM |
| **P00829** | *ATP5F1B* | ATP synthase subunit beta, mitochondrial | GLOB | CS, PM |
| **F1MWJ0** | *CDSN* | Corneodesmosin | SP | PM |
| **Q01107** | *DSC1* | Desmocollin-1 | SP+TM | PM |
| **Q03763** | *DSG1* | Desmoglein-1 | SP+TM | PM |
| **E1B9P4** | *ELSPBP1* | Epididymal sperm binding protein 1 | SP | CS |
| **O18956** | *ENTPD1* | Ectonucleoside triphosphate diphosphohydrolase 1 | TM | PM |
| **Q32LB5** | *GLIPR1L1* | GLIPR1-like protein 1 | SP | PM, ES |
| **Q76LV2** | *HSP90AA1* | Heat shock protein HSP 90-alpha | GLOB | CS, PM |
| **Q27975** | *HSPA1A* | Heat shock 70 kDa protein 1A | GLOB | PM |
| **P0CB32** | *HSPA1L* | Heat shock 70 kDa protein 1-like | GLOB | PM |
| **P34933** | *HSPA2* | Heat shock-related 70 kDa protein 2 | GLOB | CS, PM |
| **Q0VCX2** | *HSPA5* | Endoplasmic reticulum chaperone BiP | SP | CS, PM, ES |
| **P19120** | *HSPA8* | Heat shock cognate 71 kDa protein | GLOB | PM, ES |
| **P31081** | *HSPD1* | 60 kDa heat shock protein, mitochondrial | GLOB | CS, PM, ES |
| **P24627** | *LTF* | Lactotransferrin | SP | CS, PM, ES |
| **P05307** | *P4HB* | Protein disulfide-isomerase | SP | CS, PM, ESPM |
| **P38657** | *PDIA3* | Protein disulfide-isomerase A3 | SP | CS, PM, ES |
| **F1MK52** | *PROM1* | Prominin 1 | SP+TM | CS, PM, ES, ICPM |
| **E1BI52** | *PRSS42* | Serine protease 42 | SP | PM, ES, ACPM |
| **Q3MHW6** | *SLC16A1* | Monocarboxylate transporter 1 | TM | PM, ICPM |
| **Q2YDD9** | *SLC25A31* | ADP/ATP translocase 4 | TM | PM |
| **P58352** | *SLC2A3* | Solute carrier family 2, facilitated glucose transporter member 3 | TM | PM, ES, ICPM |
| **F1MI43** | *SPA17* | Sperm surface protein Sp17 | GLOB | ESPM |
| **Q32PB3** | *SPACA4* | Sperm acrosome membrane-associated protein 4 | SP | PM |
| **F1MTV1** | *SPAM1* | Hyaluronidase | SP | PM |
| **P54281** | | Calcium-activated chloride channel regulator 1 | SP | ICPM |
| **AOA3Q1MQE3** | | Uncharacterized protein | SP+TM | PM, ESPM |
| **G5E622** | | Uncharacterized protein | SP+TM | PM, ESPM |

Among these 39 proteins is ADAM2, one of the proteins significantly more expressed in BX samples than in BY samples. Also, 19 of them were identified in BX samples but not in BY samples, making them potential valid targets of X-sperm. These are the ADAM2, the Phospholipid-transporting ATPase (ATP11C), the Plasma membrane calcium-transporting ATPase 4 (PMCA4), the Desmoglein-1 (DG1), the Monocarboxylate transporter 1 (MCT1), the ATP synthase subunit beta, mitochondrial (ATP5F1B), the 60 kDa heat shock protein, mitochondrial (CPN60), the Heat shock-related 70 kDa protein 2 (HSP70.3), the Endoplasmic reticulum chaperone BiP (BiP), the Sodium/potassium-transporting ATPase subunit beta-3 (ATPB-3), Hyaluronidase (SPAM1), Alkaline phosphatase tissue-nonspecific isozyme (TNAP), Heat shock 70 kDa protein 1L (HSPA1L), Heat shock 70 kDa protein 8 (HSPA8), Heat shock 70 kDa protein 1 (HSP70.1), GLIPR1-like protein 1 (GLIPR1L1), Sperm acrosome membrane-associated protein 4 (SPACA4), and two uncharacterized proteins (disintegrins). Among these 19 proteins, the first 5 have the highest potential for application in sperm sexing techniques as they are predicted to be TM proteins with at least one TMR and were identified in 2 or 3 replicates of the BX samples. A list of the respective proteins in humans, rabbits, pigs, sheep, and horses with the percentage of similarity between their protein sequences is available in Table 15.

**Table 15. The 5 potential bovine X-semen targets possibly accessible from the cell surface identified through the analysis of the mass spectrometry data in at least 2 replicas of BX samples and not in BY samples. Each target is associated with its respective Reference Sequence (RefSeq) identifier, the percentage of protein sequence similarity with that of orthologs from other species, and the encoding chromosome for the respective proteins and species. Orthology was determined based on the NCBI database, local synteny, and/or Ensembl BioMart tool information.**

| **Species** | | **Proteins** | | | | |
|---|---|---|---|---|---|---|
| | | **ADAM2** | **ATP11C** | **ATP2B4** | **DG1** | **MCT1** |
| **Bovine** | RefSeq (Chr) | XP_0248419 56 (Chr 27) | NP_0012800 29 (Chr X) | XP_0248318 10 (Chr 16) | NP_776470 (Chr 24) | XP_0597385 38 (Chr 3) |
| **Human** | Similarity (%) | 60.5 | 92.5 | 89.8 | 81 | 85.2 |
| | RefSeq (Chr) | XP_0542162 15 (Chr 8) | XP_0541828 60 (Chr X) | NP_001675 (Chr 1) | NP_001933 (Chr 18) | XP_0541944 29 (Chr 1) |
| **Rabbit** | Similarity (%) | 59.3 | 91.6 | 89.6 | 80.6 | 87.6 |
| | RefSeq (Chr) | XP_0516847 94 (Chr 2) | XP_0516875 39 (U) | XP_0027176 26 (Chr 16) | XP_00271352 9 (Chr 9) | XP_0517121 13 (Chr 13) |
| **Pig** | Similarity (%) | 66.07 | 92.1 | 96.3 | 85 | 91.2 |
| | RefSeq (Chr) | NP_999122 (Chr 15) | XP_0209358 53 (ChrX) | XP_0209188 54 (Chr 9) | NP_00103061 2 (Chr 6) | XP_0209441 10 (Chr 4) |
| **Sheep** | Similarity (%) | 81.3 | 96.9 | 97.6 | 94.9 | 97.4 |
| | RefSeq (Chr) | XP_0278184 44 (Chr 26) | XP_0602642 32 (ChrX) | XP_0602522 28 (Chr 12) | XP00402050 2 (Chr 23) | XP_0602688 67 (Chr 1) |
| **Horse** | Similarity (%) | 68 | 90.1 | 93.7 | 82.6 | 89 |
| | RefSeq (Chr) | XP_0234865 57 (Chr 27) | XP_0234895 20 (ChrX) | XP_0014883 33 (Chr 5) | XP_00561287 5 (Chr 8) | XP_0234959 65 (Chr 5) |

Among these five proteins, ADAM2 exhibits the lowest sequence similarities between bovine and the other species. The quantified bovine proteins were also cross-referenced with the bovine X chromosome proteome and at least 5 are already associated with the X chromosome. Those are the ATP11C, the Plastin-3 (PLS3), the A-kinase anchoring protein 4 (AKAP4), the FUN14 domain-containing protein 2 (FUNDC2), and the Serine/threonine-protein phosphatase with EF-hands (PPEF1).

To extend the comparison, the quantified proteins were also cross-referenced with the human X chromosome proteome. A total of 6 proteins were found to be common. Apart from the 5 already mentioned (ATP11C, PLS3, AKAP4, FUNDC2, and PPEF1), the other common protein is the Cylicin-1 (CYLC1), defined as unplaced in the bovine proteome according to UniProt and as encoded by the X chromosome according to the NCBI database. However, only ATP11C and PLS3 are among the 52 proteins of interest determined, with ATP11C being part of those possibly accessible from the cell surface both in bovine and humans. Moreover, ATP11C is part of the 11 proteins found in BX samples but not in BY samples. The others, at least according to the available data, are not associated with the plasma membrane. ATP11C has also the potential to be a target in both humans and rabbits, as indicated by the cross-species analysis in Example 1.

### Example 4. Correlating rabbit and bovine quantified proteins

The list of quantified proteins in rabbit semen samples was also compared to the list of quantified proteins in bovine samples for new insights into possible common targets. A total of 75 proteins with a common gene name were identified. According to the Ensembl database, 66 proteins exhibit orthology between both species, with 48 having high confidence and 18 having low confidence. Among the 18 having a low confidence orthology, 13 were also identified as orthologs in the NCBI database. Additionally, 7 other proteins are recognized as orthologs solely in the NCBI database. In total, 73 proteins have some form of information available either in the Ensembl database or NCBI database, indicating that they share orthology between bovines and rabbits (Table 16).

**Table 16. List of the 73 quantified proteins shared between the rabbit and bovine sperm proteomes obtained in this study. GO terms. Gene Ontology terms related to the plasma membrane, cell surface, or extracellular space, PM. Plasma Membrane, CS. Cell surface, ESPM. External side of plasma membrane, ECESPM. Extrinsic component of external side of plasma membrane, ACPM. Anchored component of plasma membrane, ACESPM. Anchored component of external side of plasma membrane, ICPM. Integral component of plasma membrane, ES. Extracellular space. TM. Transmembrane protein, SP. signal peptide, SP+TM. Transmembrane protein with signal peptide, GLOB. Globular protein, BX. Bovine X-semen, BV. Bovine Y-semen, x. Identified in BX but not in BY, *Associated with the PM both in rabbit and bovine sperm samples, **Associated with the PM and possibly accessible from the CS both in rabbit and bovine sperm samples.**

| **Protein ID** | | **Protein name** | **Gene name** | **GO terms** | | **Topology** | | **identified in BX but not in BY** | **Interest** |
|---|---|---|---|---|---|---|---|---|---|
| **Rabbit** | **Bovine** | | | **Rabbit** | **Bovine** | **Rabbit** | **Bovine** | | |
| G1T6L2 | F1MB32 | Alpha-2-macroglobulin like 1 | *A2ML1* | ES | ES | SP | SP | | |
| G1TMV8 | A0A3Q1LJ59 | Uncharacterized protein | *Abca14* | | | TM | TM | | |
| G1SS41 | P48818 | Very long-chain specific acyl-CoA dehydrogenase, mitochondrial | *ACADVL* | | | GLOB | GLOB | x | |
| **P12822** | **F1MQJ0** | **Angiotensin-converting enzyme** | ***ACE*** | **CS, PM, ESPM, ES, ICPM** | **CS, PM, ESPM, ES** | **SP+TM** | **SP+TM** | | ** |
| G1TUX2 | P20004 | Aconitate hydratase, mitochondrial | *ACO2* | | | GLOB | GLOB | | |
| P48038 | G5E5C6 | Acrosin | *ACR* | | | SP | SP | x | |
| G1SKA8 | E1B7S8 | Acrosin-binding protein | *ACRBP* | | | SP | SP | x | |
| **P29751** | P60712 | Actin, cytoplasmic 1 | *ACTB* | **PM** | **PM** | GLOB | GLOB | | * |
| G1TJB2 | Q32KZ2 | Actin-like protein 7A | *ACTL7A* | | | GLOB | GLOB | | |
| G1U4K9 | E1B7X2 | Uncharacterized protein | *ACTL10* | | | GLOB | GLOB | | |
| G1TUC8 | A5D7D1 | Alpha-actinin-4 | *ACTN4* | | | GLOB | GLOB | | |
| Q28660 | O77780 | Disintegrin and metalloproteinase domain-containing protein 2 | *ADAM2* | | CS | SP+TM | SP+TM | x | |
| G1SGI7 | O77797 | A-kinase anchor protein 3 | *AKAP3* | | | GLOB | GLOB | x | |
| **G1SLU6** | **P09487** | **Alkaline phosphatase, tissue-nonspecific isozyme** | ***ALPL*** | **PM, ES, ACPM** | **PM, ES, ACPM** | GLOB | SP | x | ** |
| **P51662** | **P46193** | **Annexin A1** | ***ANXA1*** | **CS, PM, ESPM, ES, ECESPM** | **CS, PM, ESPM, ES, ECESPM** | **GLOB** | **GLOB** | | ** |
| G1TE72 | E1B8N5 | Sodium/potassium-transporting ATPase subunit alpha | *ATP1A4* | | PM | TM | TM | | |
| **Q9GLC3** | **Q3T0C6** | **Sodium/potassium-transporting ATPase subunit beta-3** | ***ATP1B3*** | **PM, ICPM** | **PM, ICPM** | **TM** | **TM** | x | ** |
| **G1SKT4** | P19483 | ATP synthase subunit alpha, mitochondrial | *ATP5F1A* | **PM** | **PM** | GLOB | GLOB | x | * |
| **G1SQA8** | **P00829** | **ATP synthase subunit beta, mitochondrial** | ***ATP5F1B*** | **CS, PM** | **CS, PM** | **GLOB** | **GLOB** | x | ** |
| A0A5F9DTX3 | Q32L61 | Calcium binding tyrosine phosphorylation regulated | *CABYR* | | | GLOB | GLOB | | |
| **P62160** | P62157 | Calmodulin | *CALM2* | **PM, ICPM** | **PM, ICPM** | GLOB | GLOB | x | * |
| U3KNA0 | A0A3Q1M0A1 | Cornulin | *CRNN* | | | GLOB | GLOB | | |
| U3KMZ2 | P35662 | Cylicin-1 | *CYLC1* | | | GLOB | GLOB | | |
| G1T7V5 | F1N206 | Dihydrolipoyl dehydrogenase | *DLD* | | | GLOB | GLOB | x | |
| **G1T4V7** | E1BKT9 | Desmoplakin | *DSP* | **PM** | **PM** | GLOB | GLOB | | * |
| A0A5F9CNK2 | Q9XSJ4 | Alpha-enolase | *ENO1* | | PM | GLOB | GLOB | x | |
| G1T3U7 | O18956 | Ectonucleoside triphosphate diphosphohydrolase 1 | *ENTPD1* | | PM | TM | TM | | |
| G1T7M5 | E1B9N6 | Envoplakin | *EVPL* | PM | | GLOB | GLOB | | |
| G1SZ91 | P55052 | Fatty acid-binding protein 5 | *FABPS* | ES | ES | GLOB | GLOB | x | |
| G1TTS1 | Q8MJN0 | FUN14 domain-containing protein 2 | *FUNDC2* | | | TM | TM | x | |
| A0A5F9C381 | Q2KJE5 | Glyceraldehyde-3-phosphate dehydrogenase, testis-specific | *GAPDHS* | | | GLOB | GLOB | x | |
| G1SHD8 | F1N7X7 | Family with sequence similarity 71 member B | *GARIN3* | | | GLOB | GLOB | x | |
| A0A5F9CX95 | Q32LB5 | GLIPR1-like protein 1 | *GLIPR1L1* | | PM, ES | SP | SP | x | |
| G1SRI8 | F1MZV1 | Hexokinase | *HK1* | | | GLOB | GLOB | | |
| **A0A5F9CS69** | **Q76LV2** | **Heat shock protein HSP 90-alpha** | ***HSP90AA1*** | **CS, PM** | **CS, PM** | **GLOB** | **GLOB** | | ** |
| **G1T1V9** | **P34933** | **Heat shock-related 70 kDa protein 2** | ***HSPA2*** | **CS, PM** | **CS, PM** | **GLOB** | **GLOB** | x | ** |
| **A0A5F9C804** | **Q0VCX2** | **Endoplasmic reticulum chaperone BiP** | ***HSPAS*** | **CS, PM, ES** | **CS, PM, ES** | **SP** | **SP** | x | ** |
| A0A5F9C6Y7 | P19120 | Heat shock cognate 71 kDa protein | *HSPA8* | | PM, ES | GLOB | GLOB | x | |
| G1SRF7 | Q3ZCH0 | Stress-70 protein, mitochondrial | *HSPA9* | | | GLOB | GLOB | x | |
| **G1T3Y8** | **P31081** | **60 kDa heat shock protein, mitochondrial** | ***HSPD1*** | **CS, PM, ES** | **CS, PM, ES** | **GLOB** | **GLOB** | x | ** |
| G1TSX5 | P61603 | 10 kDa heat shock protein, mitochondrial | *HSPE1* | | | GLOB | GLOB | x | |
| G1SI20 | Q9XSG3 | Isocitrate dehydrogenase [NADP] cytoplasmic | *IDH1* | | | GLOB | GLOB | | |
| A0A5F9DQ55 | Q04467 | Isocitrate dehydrogenase [NADP], mitochondrial | *IDH2* | | | GLOB | GLOB | x | |
| G1TZK5 | Q3T056 | L-lactate dehydrogenase A-like 6B | *LDHAL6B* | | | GLOB | GLOB | x | |
| G1TIS1 | E1BNS9 | L-lactate dehydrogenase | *LDHC* | | | GLOB | GLOB | | |
| A0A5F9CN88 | Q32LG3 | Malate dehydrogenase, mitochondrial | *MDH2* | | | GLOB | GLOB | x | |
| U3KMI7 | F1ML59 | 5'-nucleotidase, cytosolic IB | *NT5C18* | | | GLOB | GLOB | x | |
| A0A5F9CLF0 | Q2T9U2 | Outer dense fiber protein 2 | *ODF2* | | | GLOB | GLOB | | |
| **P21195** | **P05307** | **Protein disulfide-isomerase** | ***P4HB*** | **CS, PM, ESPM** | **CS, PM, ESPM** | **SP** | **SP** | | ** |
| **B7NZF1** | **P38657** | **Protein disulfide-isomerase A3** | ***PDIA3*** | **CS, PM, ES** | **CS, PM, ES** | **SP** | **SP** | | ** |
| Q8MK67 | P13696 | Phosphatidylethanolamine-binding protein 1 | *PEBP1* | | | GLOB | GLOB | x | |
| P47859 | A0A3Q1NJ70 | ATP-dependent 6-phosphofructokinase | *PFKP* | | | GLOB | GLOB | x | |
| G1T0I5 | F1MFA3 | Presequence protease, mitochondrial | *PITRM1* | | | GLOB | GLOB | x | |
| P11974 | A5D984 | Pyruvate kinase | *PKM* | | | GLOB | GLOB | x | |
| **G1TRY5** | A7E3Q8 | Plastin-3 | *PLS3* | **PM** | **PM** | GLOB | GLOB | | * |
| G1T005 | F1MBQ1 | Serine/threonine-protein phosphatase with EF-hands | *PPEF1* | | | GLOB | GLOB | x | |
| G1T1G8 | A0A3Q1LVG3 | Periplakin | *PPL* | | | GLOB | GLOB | | |
| G1SQ02 | Q5E947 | Peroxiredoxin 1 | *PRDX1* | | | GLOB | GLOB | x | - |
| **A0A5F9CHF3** | **F1MK52** | **Prominin 1** | ***PROM1*** | **CS, PM, ES, ICPM** | **CS, PM, ES, ICPM** | **SP+TM** | **SP+TM** | | ** |
| Q01971 | A0A3Q1MHX8 | RAB2A, member RAS onco family | *RAB2A* | | | GLOB | GLOB | x | |
| P24480 | F1MX83 | Protein S100 | *S100A11* | | | GLOB | GLOB | | |
| A0A5F9DDC1 | A0A3Q1N181 | Sciellin | *SCEL* | | | GLOB | GLOB | | |
| **G1TCC1** | **Q3MHW6** | **Monocarboxylate transporter 1** | ***SLC16A1*** | **PM, ICPM** | **PM, ICPM** | **TM** | **TM** | x | ** |
| **Q9XSC2** | **P58352** | **Solute carrier family 2, facilitated glucose transporter member 3** | ***SLC2A3*** | **ICPM** | **PM, ES, ICPM** | **TM** | **TM** | | ** |
| G 1T9T8 | Q2YDD9 | ADP/ATP translocase 4 | *SLC25A31* | | PM | TM | TM | | |
| **P36425** | **F1MI43** | **Sperm surface protein Sp17** | ***SPA17*** | **CS, PM, ESPM** | **ESPM** | **GLOB** | **GLOB** | | ** |
| G1T4H1 | Q2YDG7 | Sperm acrosome membrane-associated protein 1 | *SPACA1* | | | SP+TM | SP+TM | | |
| **G1T6C6** | **Q32PB3** | **Sperm acrosome membrane-associated protein 4** | ***SPACA4*** | **PM** | **PM** | **SP** | **SP** | x | ** |
| **G1T1B3** | **F1MTV1** | **Hyaluronidase** | ***SPAM1*** | **CS, PM, ESPM** | **PM** | **SP** | **SP** | x | ** |
| A0A5F9CAY1 | E1BLK7 | Mitochondria-eating protein | *SPATA18* | | | GLOB | GLOB | | |
| A0A5F9C3S9 | F1MKF8 | Sulfide quinone oxidoreductase | *SQOR* | | | GLOB | GLOB | x | |
| G1SDY5 | P63103 | 14-3-3 protein zeta/delta | *YWHAZ* | ES | ES | GLOB | GLOB | | |
| A0A5F9DF03 | A0A3Q1MFF0 | Zona pellucida binding protein | *ZPBP* | | | GLOB | SP | | |

Of those, 22 proteins were considered to be of interest (linked to the plasma membrane) in both rabbit and bovine samples, of which 17 proteins were considered to be possibly accessible from the cell surface in both species (Table 16). Among those 17 proteins, 9 were identified in BX samples and not in BY samples. Out of these 9 proteins, 7 were identified in 2 or more replicates of BX samples (ATP5F1B, HSPA70.3, BiP, CPN60, MCT1, SPACA4, and SPAM1) and 2 were only identified in 1 replicate (ATPB-3 and TNAP). In terms of the presence of a transmembrane domain, only ATPB-3 (TMR=1) and MCT1 (TMR=12) are predicted to have at least one. Hence, MCT1 is among the most promising candidate proteins for further exploration in sperm sexing applications.

Moreover, 4 common proteins that were considered possible targets in bovine samples due to their cellular localization (ADAM2, ATP1A4, ENTPD1, and SLC25A31) were equally predicted to be TM proteins in rabbits. The protein ADAM2 was considered the most promising of the 4 proteins for sperm sexing, since ENTPD1 was only found in BU samples, and ATP1A4 and SLC25A31 were found both in BX and BY samples. Therefore, ADAM2 may be a possible common rabbit and bovine target for sperm sexing.

Also promising for application in sperm sexing, another 4 common proteins stood out (EN01, HSPA8, HSPA1L, and GLIPR1L1). ENO1 was associated with the GO term 'plasma membrane' in bovine samples, and HSPA8, HSPA1L, and GLIPR1L1 were considered potential targets in bovines. They were also associated with the plasma membrane and their protein sequences were predicted to be located outside the cell. Moreover, all of them were identified in BX samples but not in BY samples. In rabbits, none of them had a GO term associated with the plasma membrane according to the available information, but there is a chance for them to be localized also in the plasma membrane in rabbits.

It is important to note that not being part of the plasma membrane, does not exclude the possibility of using those proteins for sperm sexing depending on their cellular function. Proteins related to sperm motility or ATP production, for example, that are specific of X- or Y-sperm can also be considered. By interfering with their function, namely by inhibiting them, sperm motility would possibly only be affected in one of the spermatozoa types. Also, if a protein was not associated with the plasma membrane and/or cell surface (namely because some are still uncharacterized) or is still unplaced and not associated with the sex chromosomes proteome, do not necessarily mean that it is not a protein of interest, since this approach highlights the information that is currently available. However, a kit targeting cell surface proteins was used and, therefore, the actual number of potential targets may be much higher. The bioinformatic analysis does not indicate that proteins are not present in the cellular localization of interest, only that they have not yet been described as such in terms of Gene Ontology terms.

### REFERENCES

[1] Nations, U World population to reach 8 billion on 15 November 2022 | United Nations. https://www.un.org/en/desa/world-population-reach-8-billion-15-november-2022, accessed on 2023-01-30.
[2] Godfray, H. C. J.; Aveyard, P.; Garnett, T.; Hall, J. W.; Key, T. J.; Lorimer, J.; Pierrehumbert, R. T.; Scarborough, P.; Springmann, M.; Jebb, S. A. Meat Consumption, Health, and the Environment. Science 2018, 361 (6399). https://doi.org/10.1126/SCIENCE.AAM5324.
[3] Henchion, M.; Moloney, A. P.; Hyland, J.; Zimmermann, J.; McCarthy, S. Review: Trends for Meat, Milk and Egg Consumption for the next Decades and the Role Played by Livestock Systems in the Global Production of Proteins. Animal 2021, 15, 100287. https://doi.org/10.1016/J.ANIMAL.2021.100287.
[4] Food and Agriculture Organization of the United Nations (FAO). FAOSTAT. https://www.fao.org/faostat/en/ridata/QCL (accessed on 2023-01-30).
[5] Adesogan, A. T.; Havelaar, A. H.; McKune, S. L.; Eilitta, M.; Dahl, G. E. Animal Source Foods: Sustainability Problem or Malnutrition and Sustainability Solution? Perspective Matters. Glob Food Sec 2020, 25, 100325. https://doi.org/10.1016/J.GFS.2019.100325.
[6] Singh, B.; Mal, G; Gautam, S. K.; Mukesh, M. Revolutionary Reproduction Biotechnologies in Livestock: An Overview. Advances in Animal Biotechnology 2019, 83-96. https://doi.org/10.1007/978-3-030-21309-1_8.
[7] Garner, D. L.; Seidel, G. E. History of Commercializing Sexed Semen for Cattle. Theriogenology 2008, 69 (7), 886-895. https://doi.org/10.1016/J.THERIOGENOLOGY.2008.01.006.
[8] Moore, S. G.; Hasler, J. F. A 100-Year Review: Reproductive Technologies in Dairy Science. J Dairy Sci 2017, 100 (12), 10314-10331. https://doi.org/10.3168/jds.2017-13138.
[9] Seidel, G. E.; Garner, D. L. Current Status of Sexing Mammalian Spermatozoa. Reproduction 2002, 124 (6), 733-743. https://doi.org/10.1530/reprod/124.6.733.
[10] Johnson, L. A. Sexing Mammalian Sperm for Production of Offspring: The State-of-the-Art. Anim Reprod Sci 2000, 60-61, 93-107. https://doi.org/10.1016/50378-4320(00)00088-9.
[11] Garner, D. L.; Evans, K. M.; Seidel, G. E. Sex-Sorting Sperm Using Flow Cytometry/Cell Sorting. 2013, 279-295. https://doi.org/10.1007/978-1-62703-038-026.
[12] Ellis, P. J. I.; Yu, Y; Zhang, S. Transcriptional Dynamics of the Sex Chromosomes and the Search for Offspring Sex-Specific Antigens in Sperm. Reproduction 2011, 142 (5), 609-619. https://doi.org/10.1530/REP-11-0228.
[13] Seidel, G. E. Update on Sexed Semen Technology in Cattle. Animal 2014, 8 (SUPPL. 1), 160-164. https://doi.org/10.1017/S1751731114000202.
[14] Álvarez-Gallardo, H.; Kjelland, M. E.; Pérez-Martínez, M.; Villaseñor-González, F.; Romo-García, S. Evaluation of Novel SexedULTRA-4M Technology for in Vitro Bovine Embryo Production. Anim Reprod 2022, 19 (1). https://doi.org/10.1590/1984-3143-AR2022-0018.
[15] Thomas, J. M.; Locke, J. W. C.; Bonacker, R. C.; Knickmeyer, E. R.; Wilson, D. J.; Vishwanath, R.; Arnett, A. M.; Smith, M. F.; Patterson, D. J. Evaluation of SexedULTRA 4MTM Sex-Sorted Semen in Timed Artificial Insemination Programs for Mature Beef Cows. Theriogenology 2019, 123, 100-107. https://doi.org/10.1016/J.THERIOGENOLOGY.2018.09.039.
[16] Mothé, G; Scott, C.; Sicherle, C. C.; Guaitolini, C. R.; Dell'aqua, C.; Malossi, C.; Araújo-Júnior, J. P.; de Souza, F. F. Sperm Sexing with Density Gradient Centrifugation in Dogs. Anim Reprod Sci 2018, 199, 84-92. https://doi.org/10.1016/j.anireprosci.2018.11.003.
[17] Lush, J. L. The Possibility of Sex Control by Artificial Insemination with Centrifuged Spermatozoa. J Agric Res 1925.
[18] Gordon, M. J. CONTROL OF SEX RATIO IN RABBITS BY ELECTROPHORESIS OF SPERMATOZOA. Proc Natl Acad Sci U S A 1957, 43 (10), 913-918. https://doi.org/10.1073/PNAS.43.10.913.
[19] Zavos, P. M. Sperm Separation Attempts via the Use of Albumin Gradients in Rabbits. Theriogenology 1985, 23 (6), 875-879. https://doi.org/10.1016/0093-691X(85)90005-6.
[20] Copello, M.; Perez, A.; Marquez, S.; Sansinena, M.; Copello, M.; Perez, A.; Marquez, S.; Sansinena, M. 202 SEX PRE-SELECTION IN RABBITS: AN ATTEMPT TO SKEW OFFSPRING SEX THROUGH PERCOLL AND SWIM-UP SPERM PREPARATION TECHNIQUES. Reprod Fertil Dev 2011, 24 (1), 213-213. https://doi.org/10.1071/RDV24N1AB202.
[21] de Luca, A. C.; Managó, S.; Ferrara, M. A.; Rendina, I.; Sirleto, L.; Puglisi, R.; Balduzzi, D.; Galli, A.; Ferraro, P.; Coppola, G. Non-Invasive Sex Assessment in Bovine Semen by Raman Spectroscopy. Laser Phys Lett 2014, 11 (5), 055604. https://doi.org/10.1088/1612-2011/11/5/055604.
[22] Katigbak, R. D.; Turchini, G. M.; de Graaf, S. P.; Kong, L.; Dumée, L. F. Review on Sperm Sorting Technologies and Sperm Properties toward New Separation Methods via the Interface of Biochemistry and Material Science. Adv Biosyst 2019, 3 (9), 1900079. https://doi.org/10.1002/ADBI.201900079.
[23] ABS Global. Sexcel® Sexed Genetics - ABS Global Australia. https://www.absglobal.com/au/dairy/sexcel-sexed-genetics/, accessed on 2022-09-01.
[24] Zuidema, D.; Kerns, K.; Sutovsky, P. An Exploration of Current and Perspective Semen Analysis and Sperm Selection for Livestock Artificial Insemination. Animals 2021, 11 (12), 3563. https://doi.org/10.3390/ani11123563
[25] Perry, G. A.; Walker, J. A.; Rich, J. J. J.; Northrop, E. J.; Perkins, S. D.; Beck, E. E.; Sandbulte, M. D.; Mokry, F. B. Influence of SexcelTM (Gender Ablation Technology) Gender-Ablated Semen in Fixed-Time Artificial Insemination of Beef Cows and Heifers. Theriogenology 2020, 146, 140-144. https://doi.org/10.1016/J.THERIOGENOLOGY.2019.11.030.
[26] Yadav, S. K.; Gangwar, D. K.; Singh, J.; Tikadar, C. K.; Khanna, V. V.; Saini, S; Dholpuria, S; Palta, P.; Manik, R. S.; Singh, M. K.; Singla, S. K. An Immunological Approach of Sperm Sexing and Different Methods for Identification of X- and Y-Chromosome Bearing Sperm. Vet World 2017, 10 (5), 498-504. https://doi.org/10.14202/VETWORLD.2017.498-504.
[27] Quelhas, J; Santiago, J.; Matos, B.; Rocha, A.; Lopes, G; Fardilha, M. Bovine Semen Sexing: Sperm Membrane Proteomics as Candidates for Immunological Selection of X- and Y-Chromosome-Bearing Sperm. Vet Med Sci 2021, 7 (5), 1633-1641. https://doi.org/10.1002/VMS3.540.
[28] Umehara,T.;Tsujita, N.; Shimada, M. Activation of Toll-like Receptor 7/8 Encoded by the X Chromosome Alters Sperm Motility and Provides a Novel Simple Technology for Sexing Sperm. PLoS Biol 2019, 17 (8). https://doi.org/10.1371/JOURNAL.PBIO.3000398.
[29] Heo, Y.-T.; Kim, D.-G.; Uhm, S. Analysis of sex Ratio on Bovine in Vitro Fertilized Embryos Using Sex Determination Kit Treated Sperm. Journal of Embryo Transfer 2018, 33 (3), 169-175.
[30] Nuri Science Inc. Animal Sperm Sexing. http://www.nurisci.com/eng/specification.php, accessed on 2022-04-21.
[31] EMLAB Genetics. Product Gallery. United States. https://www.emlabgenetics.com/shop, accessed on 2023-02-07.
[32] UP000001811, UniProt database. https://www.uniprot.org/proteomes/UP000001811
[33] UP000005640, UniProt database https://www.uniprot.org/proteomes/UP000005640
[34] ID mapping tool of the UniProt database. https://www.uniprot.org/id-mapping
[35] Ensembl BioMart tool (Dataset Rabbit genes - OryCun 2.0, filter "Transcript stable ID(s)"). https://www.ensembl.org/biomart/martview/803b80deb7176aa68139fb172542e22e, accessed on 2022-06-01
[36] EggNOG-mapper v2.1.7 tool. http://eggnog-mapper.embl.de/, accessed on 2022-06-20
[37] DeepTMHMM. https://dtu.biolib.com/DeepTMHMM/, accessed on 2022-06-24
[38] Hallgren, J.; Tsirigos, K.D.; Pedersen, MD; Almagro Armenteros, J.J.; Marcatili, P.; Nielsen, H.; Krogh, A.; Winther, O. DeepTMHMM Predicts Alpha and Beta Transmembrane Proteins Using Deep Neural Networks. BioRxiv 2022, 2022-2024.
[39] GitHub. https://github.com/PATRICIAPINHO/Gene-Ontology-analysis
[40] AmiGO 2 GENEONTOLOGY web application. http://amigo.geneontology.org/amigo, accessed on 2022-07-28
[41] Sangar, V.; Blankenberg, DJ; Altman, N.; Lesk, A.M. Quantitative Sequence-Function Relationships in Proteins Based on Gene Ontology. BMC Bioinformatics 2007, 8, 1-15.
[42] Joshi, T.; Xu, D. Quantitative Assessment of Relationship between Sequence Similarity and Function Similarity. BMC Genomics 2007, 8, 1-10.
[43] Higdon, R.; Louie, B.; Kolker, E. Modeling Sequence and Function Similarity between Proteins for Protein Functional Annotation. In Proceedings of the Proceedings of the 19th ACM International Symposium on High Performance Distributed Computing; 2010; pp. 499-502.
[44] Queirós, B. Master's dissertion 'Impact of Sperm Protein Translation on Motility', University of Aveiro: Aveiro, 2023.
[45] Zhang X, Smits AH, Van Tilburg GBA, Ovaa H, Huber W, Vermeulen M. Proteome-wide identification of ubiquitin interactions using UbIA-MS. Nat Protoc (2018) 13:530-550. https://doi.org/10.1038/nprot.2017.147
[46] Ritchie ME, Phipson B, Wu DI, Hu Y, Law CW, Shi W, Smyth GK. Limma powers differential expression analyses for RNA-sequencing and microarray studies. Nucleic Acids Res (2015) 43:e47-e47. https://doi.org/10.1093/nar/gkv007
[47] Butler ML, Bormann JM. Weaber RL, Grieger DM, Rolf MM. Selection for Bull Fertility: A Review Transl Anim Sci (2020) 4:423-441. http://doi.org/10.1093/tas/txz174
[48] UP000009136, UniProt database https://www.uniprot.org/proteomes/UP000009136
[49] Ensembl BioMart https://www.ensembl.org/biomart/martview/a6763ed18bfc217c68f09070ab50e1f1, accessed on 26 September 2023.

## Claims

1. Method for *in vitro* identification of sperm sexing protein targets, preferably membrane targets, comprising the steps of:
collecting a semen sample from a mammal, preferably a human, rabbit, cattle, pig, sheep or horse; extracting proteins from said semen sample;
carrying out liquid chromatography with tandem mass spectrometry in said proteins, optionally incubating said proteins with PGNase and optionally purifying and cleaning said proteins;
performing a bioinformatic analysis of said sample to identify potential protein candidates for an immunologically based sperm sexing technique.

2. Method according to the previous claim, wherein the protein targets have at least 95% homology with one or a combination of the following: SEQ ID No. 1 (Adhesion G protein-coupled receptor G2 (ADGRG2)), SEQ ID No. 2 (Renin receptor (ATP6AP2)), SEQ ID No. 3 (V-set and immunoglobulin domain-containing protein 1 (VSIG1)), SEQ ID No. 4 (B-cell receptor-associated protein (BCAP31)), SEQ ID No. 5 (membrane-associated progesterone receptor component 1 (PGRMC1)), SEQ ID No. 6 (Four and a half LIM domains 1 (FHL1)), SEQ ID No. 7 (Plastin 3 (PLS3)), SEQ ID No. 8 (RNA helicase (DDX3X)), SEQ ID No. 9 (Disintegrin and metalloproteinase domain-containing protein 2 (ADAM 2)), SEQ ID No. 10 (Phospholipid-transporting ATPase IG (ATP11C)), SEQ No. 11 (Desmoglein-1 (DG1)), SEQ ID No. 12 (Monocarboxylate transporter 1 (MCT 1)), SEQ ID No. 13 (Plasma membrane calcium-transporting ATPase 4 (PMCA4)), SEQ ID No. 14 (A-kinase anchoring protein 4 (AKAP4)), SEQ ID No. 15 (Cylicin-1 (CYLC1)), SEQ ID No. 16 (FUN14 domain-containing protein 2 (FUNDC2)), SEQ ID No. 17 (Serine/threonine-protein phosphatase with EF-hands (PPEF1)), SEQ ID No. 18 (Alpha-enolase (ENO1)), SEQ ID No. 19 (GLIPR1-like protein 1 (GLIPR1L1)), SEQ ID No. 20 (Heat shock 70 kDa protein 1-like (HSPA1L)), SEQ ID No. 21 (Heat shock cognate 71 kDa protein (HSPA8)), SEQ ID No. 22 (Malate dehydrogenase, mitochondrial (MDH2)), SEQ ID No. 23 (Phosphatidylethanolamine-binding protein 1 (PEBP1)), SEQ ID No. 24 (Sodium/potassium-transporting ATPase subunit beta-3 (ATPB-3)), SEQ ID No. 25 (ATP synthase subunit beta, mitochondrial (ATP5F1B)), SEQ ID No. 26 (Heat shock-related 70 kDa protein 2 (HSP70.3)), SEQ ID No. 27 (Endoplasmic reticulum chaperone BiP (BiP)), SEQ ID No. 28 (Chaperonin 60 (CPN60)), SEQ ID No. 29 (ADAM metallopeptidase domain 20), SEQ ID No. 30 (Disintegrin and metalloproteinase domain-containing protein 1a-like), SEQ ID No. 31 (Hyaluronidase (SPAM1)), SEQ ID No. 32 (Alkaline phosphatase, tissue-nonspecific isozyme (AP-TNAP)), SEQ ID No. 33 (Heat shock 70 kDa protein 1A (HSPA1A)), and SEQ ID No. 34 (Sperm acrosome membrane-associated protein 4 (SPACA4)), preferably ATP11C, MCT1, ADAM2 and PMCA4.

3. Method according to the previous claim, wherein the protein targets have at least 98% homology with SEQ ID No 1 - 34, even more preferably having at least 99% homology with SEQ ID No 1 - 34.

4. Method according to any one of claims 1-3 for *in vitro* sperm sexing technologies, comprising a step of performing an immunoassay to identify a protein target having at least 95% homology with one or a combination of the following: SEQ ID No. 1 (Adhesion G protein-coupled receptor G2 (ADGRG2)), SEQ ID No. 2 (Renin receptor (ATP6AP2)), SEQ ID No. 3 (V-set and immunoglobulin domain-containing protein 1 (VSIG1)), SEQ ID No. 4 (B-cell receptor-associated protein (BCAP31)), SEQ ID No. 5 (membrane-associated progesterone receptor component 1 (PGRMC1)), SEQ ID No. 6 (Four and a half LIM domains 1 (FHL1)), SEQ ID No. 7 (Plastin 3 (PLS3)), SEQ ID No. 8 (RNA helicase (DDX3X)), SEQ ID No. 9 (Disintegrin and metalloproteinase domain-containing protein 2 (ADAM 2)), SEQ ID No. 10 (Phospholipid-transporting ATPase IG (ATP11C)), SEQ No. 11 (Desmoglein-1 (DG1)), SEQ ID No. 12 (Monocarboxylate transporter 1 (MCT 1)), SEQ ID No. 13 (Plasma membrane calcium-transporting ATPase 4 (PMCA4)), SEQ ID No. 14 (A-kinase anchoring protein 4 (AKAP4)), SEQ ID No. 15 (Cylicin-1 (CYLC1)), SEQ ID No. 16 (FUN14 domain-containing protein 2 (FUNDC2)), SEQ ID No. 17 (Serine/threonine-protein phosphatase with EF-hands (PPEF1)), SEQ ID No. 18 (Alpha-enolase (ENO1)), SEQ ID No. 19 (GLIPR1-like protein 1 (GLIPR1L1)), SEQ ID No. 20 (Heat shock 70 kDa protein 1-like (HSPA1L)), SEQ ID No. 21 (Heat shock cognate 71 kDa protein (HSPA8)), SEQ ID No. 22 (Malate dehydrogenase, mitochondrial (MDH2)), SEQ ID No. 23 (Phosphatidylethanolamine-binding protein 1 (PEBP1)), SEQ ID No. 24 (Sodium/potassium-transporting ATPase subunit beta-3 (ATPB-3)), SEQ ID No. 25 (ATP synthase subunit beta, mitochondrial (ATP5F1B)), SEQ ID No. 26 (Heat shock-related 70 kDa protein 2 (HSP70.3)), SEQ ID No. 27 (Endoplasmic reticulum chaperone BiP (BiP)), SEQ ID No. 28 (Chaperonin 60 (CPN60)), SEQ ID No. 29 (ADAM metallopeptidase domain 20), SEQ ID No. 30 (Disintegrin and metalloproteinase domain-containing protein 1a-like), SEQ ID No. 31 (Hyaluronidase (SPAM1)), SEQ ID No. 32 (Alkaline phosphatase, tissue-nonspecific isozyme (AP-TNAP)), SEQ ID No. 33 (Heat shock 70 kDa protein 1A (HSPA1A)), and SEQ ID No. 34 (Sperm acrosome membrane-associated protein 4 (SPACA4)), preferably ATP11C, MCT1, ADAM2 and PMCA4.

5. Use of the method of claims 1-4 in medicine, veterinary medicine and agriculture.
